# EUROPÄISCHE PATENTANMELDUNG

(11) **EP 1 956 010 A1**
(43) Veröffentlichungstag der Anmeldung: **13.08.2008**
(21) Anmeldenummer: 07101785.9
(22) Anmeldetag: 06.02.2007
(51) Int. Cl.: C07D 239/94, C07D 401/12, A61K 31/517, A61P 35/00

(54) **Bicyclische Heterocyclen, diese Verbindungen enthaltende Arzneimittel,deren Verwendung und Verfahren zu ihrer Herstellung**

(71) Anmelder: Boehringer Ingelheim Pharma GmbH & Co. KG, 55216 Ingelheim (DE)
(72) Erfinder: Die Erfindernennung liegt noch nicht vor
(74) Vertreter: Hammann, Heinz

(57) **Zusammenfassung**

Die vorliegende Erfindung betrifft bicyclische Heterocyclen der allgemeinen Formel 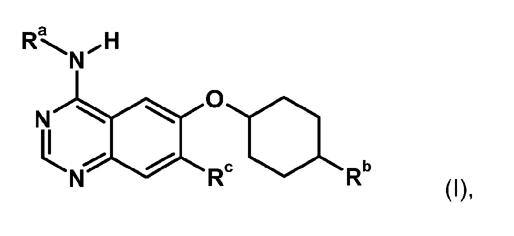 in der
R^{a}, R^{b} und R^{c} wie im Anspruch 1 definiert sind, deren Tautomere, deren Stereoisomere, deren Gemische und deren Salze, insbesondere deren physiologisch verträgliche Salze mit anorganischen oder organischen Säuren oder Basen, welche wertvolle pharmakologische Eigenschaften aufweisen, insbesondere eine Hemmwirkung auf die durch Tyrosinkinasen vermittelte Signaltransduktion, deren Verwendung zur Behandlung von Krankheiten, insbesondere von Tumorerkrankungen sowie der benignen Prostatahyperplasie (BPH), von Erkrankungen der Lunge und der Atemwege und deren Herstellung.

## Beschreibung

Gegenstand der vorliegenden Erfindung sind bicyclische Heterocyclen der allgemeinen Formel 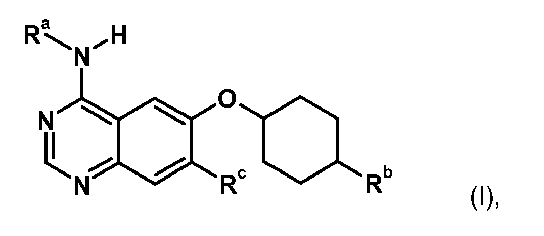 deren Tautomere, deren Stereoisomere, deren Gemische und deren Salze, insbesondere deren physiologisch verträgliche Salze mit anorganischen oder organischen Säuren und Basen, welche wertvolle pharmakologische Eigenschaften aufweisen, insbesondere eine Hemmwirkung auf die durch Tyrosinkinasen vermittelte Signaltransduktion, deren Verwendung zur Behandlung von Krankheiten, insbesondere von Tumorerkrankungen sowie der benignen Prostatahyperplasie (BPH), von Erkrankungen der Lunge und der Atemwege und deren Herstellung.

In der obigen allgemeinen Formel I bedeutet
R^{a} eine Phenyl- oder 1-Phenylethyl-Gruppe, in denen der Phenylkern jeweils durch die Reste R¹ bis R³ substituiert ist, wobei
   R¹ und R², die gleich oder verschieden sein können, jeweils ein Wasserstoff-, Fluor-, Chlor-, Brom- oder Jodatom,
   eine C₁₋₄-Alkyl-, Hydroxy-, C₁₋₄-Alkoxy-, C₂₋₃-Alkenyl- oder C₂₋₃-Alkinyl-Gruppe,
   eine Aryl-, Aryloxy-, Arylmethyl- oder Arylmethoxy-Gruppe,
   eine Heteroaryl-, Heteroaryloxy-, Heteroarylmethyl- oder Heteroarylmethoxy-Gruppe,
   eine durch 1 bis 3 Fluoratome substituierte Methyl- oder Methoxy-Gruppe oder
   eine Cyano-, Nitro- oder Amino-Gruppe, und
   R³ ein Wasserstoff-, Fluor-, Chlor- oder Bromatom oder
   eine Methyl- oder Trifluormethyl-Gruppe darstellen,
R^{b} eine Azetidin-1-yl-, Pyrrolidin-1-yl-, Piperidin-1-yl-, Homopiperidin-1-yl-, Morpholin-4-yl-, Homomorpholin-4-yl, Piperazin-1-yl-, 4-(C₁₋₄-Alkyl-carbonyl)-piperazin-1-yl-, 4-(C₁₋₄-Alkyl-sulfonyl)-piperazin-1-yl-, Homopiperazin-1-yl-, 4-(C₁₋₄-Alkyl-carbonyl)-homopiperazin-1-yl- oder 4-(C₁₋₄-Alkyl-sulfonyl)-homopiperazin-1-yl-Gruppe, die jeweils durch R⁴ mono-, di- oder trisubstituiert sein kann, wobei die Substituenten gleich oder verschieden sein können und
   R⁴ ein Fluor-, Chlor-, Brom- oder lodatom,
   eine C₁₋₄-Alkyl-, C₂₋₄-Alkenyl- oder C₂₋₄-Alkinyl-Gruppe,
   eine durch 1 bis 3 Fluoratome substituierte Methyl- oder Methoxy-Gruppe,
   eine Amino-, C₁₋₄-Alkylamino-, Di-(C₁₋₄-alkyl)amino-, C₁₋₄-Alkyl-carbonylamino-, N-(C₁₋₄-Alkyl)-C₁₋₄-alkyl-carbonylamino-, C₁₋₄-Alkyl-sulfonylamino- oder N-(C₁₋₄-Alkyl)-C₁₋₄-alkyl-sulfonylamino-Gruppe,
   eine Amino-C₁₋₄-alkyl-, C₁₋₄-Alkylamino-C₁₋₄-alkyl-, Di-(C₁₋₄-alkyl)amino-C₁₋₄-alkyl-, C₁₋₄-Alkyl-carbonylamino-C₁₋₄-alkyl-, N-(C₁₋₄-Alkyl)-C₁₋₄-alkyl-carbonylamino-C₁₋₄-alkyl-, C₁₋₄-Alkyl-sulfonylamino-C₁₋₄-alkyl- oder N-(C₁₋₄-Alkyl)-C₁₋₄-alkyl-sulfonylamino-C₁₋₄-alkyl-Gruppe,
   eine Hydroxy-, C₁₋₄-Alkyloxy- oder C₁₋₄-Alkyl-carbonyloxy-Gruppe
   eine Hydroxy-C₁₋₄-alkyl-, C₁₋₄-Alkyloxy-C₁₋₄-alkyl- oder C₁₋₄-Alkyl-carbonyloxy-C₁₋₄-alkyl-Gruppe,
   eine C₁₋₄-Alkyl-carbonyl-, Cyano-, C₁₋₄-Alkyl-oxycarbonyl-, Carboxy-, Aminocarbonyl-, C₁₋₄-Alkyl-aminocarbonyl-, Di-(C₁₋₄-alkyl)amino-carbonyl-, Pyrrolidin-1-yl-carbonyl-, Piperidin-1-yl-carbonyl-, Piperazin-1-yl-carbonyl-, 4-C₁₋₄-Alkyl-piperazin-1-yl-carbonyl- oder Morpholin-4-yl-carbonyl-Gruppe,
   eine C₁₋₄-Alkylcarbonyl-C₁₋₄-alkyl-, Cyano-C₁₋₄-alkyl-, C₁₋₄-Alkyloxycarbonyl-C₁₋₄-alkyl-, Aminocarbonyl-C₁₋₄-alkyl-, C₁₋₄-Alkylaminocarbonyl-C₁₋₄-alkyl-, Di-(C₁₋₄-alkyl)aminocarbonyl-C₁₋₄-alkyl-, Pyrrolidin-1-yl-carbonyl-C₁₋₄-alkyl-, Piperidin-1-yl-carbonyl-C₁₋₄-alkyl-, Piperazin-1-yl-carbonyl-C₁₋₄-alkyl-, 4-C₁₋₄-Alkyl-piperazin-1-yl-carbonyl-C₁₋₄-alkyl- oder Morpholin-4-yl-carbonyl-C₁₋₄-alkyl-Gruppe,
   eine C₁₋₄-Alkylsulfanyl-, C₁₋₄-Alkylsulfinyl-, C₁₋₄-Alkylsulfonyl-, Aminosulfonyl-, C₁₋₄-Alkyl-aminosulfonyl- oder Di-(C₁₋₄-alkyl)amino-sulfonyl-Gruppe,
   eine C₁₋₄-Alkylsulfanyl-C₁₋₄-alkyl-, C₁₋₄-Alkylsulfinyl-C₁₋₄-alkyl-, C₁₋₄-Alkylsulfonyl- C₁₋₄-alkyl-, Aminosulfonyl-C₁₋₄-alkyl-, C₁₋₄-Alkylaminosulfonyl-C₁₋₄-alkyl- oder Di-(C₁₋₄-alkyl)amino-sulfonyl-C₁₋₄-alkylGruppe
   darstellt, und wobei die vorstehend erwähnten Heterocylen zusätzlich durch eine Oxo-Gruppe substituiert sein können,
R^{c} ein Wasserstoffatom,
ein Fluor-, Chlor-, Brom- oder lodatom,
eine C₁₋₄-Alkylgruppe,
eine C₁₋₄-Alkylgruppe, die durch einen Rest R⁵ substituiert ist, wobei
   R⁵ eine Hydroxy-, C₁₋₃-Alkyloxy-, C₃₋₆-Cycloalkyloxy-, Amino-, C₁₋₃-Alkylamino-, Di-(C₁₋₃-alkyl)amino-, Bis-(2-methoxyethyl)-amino-, Pyrrolidin-1-yl-, Piperidin-1-yl-, Homopiperidin-1-yl-, Morpholin-4-yl-, Homomorpholin-4-yl-, 2-Oxa-5-aza-bicyclo[2.2.1]hept-5-yl-, 3-Oxa-8-aza-bicyclo[3.2.1]oct-8-yl-, 8-Oxa-3-aza-bicyclo[3.2.1]oct-3-yl-, Piperazin-1-yl-, 4-C₁₋₃-Alkyl-piperazin-1-yl-, Homopiperazin-1-yl- oder C₁₋₃-Alkyl-homopiperazin-1-yl-Gruppe oder
   eine Formylamino-, C₁₋₄-Alkylcarbonylamino-, C₁₋₃-Alkyloxy-C₁₋₃-alkyl-carbonylamino-, C₁₋₄-Alkyloxycarbonylamino-, Aminocarbonylamino-, C₁₋₃-Alkylaminocarbonylamino-, Di-(C₁₋₃-alkyl)aminocarbonylamino-, Pyrrolidin-1-ylcarbonylamino-, Piperidin-1-ylcarbonylamino-, Piperazin-1-ylcarbonylamino-, 4-C₁₋₃-Alkyl-piperazin-1-ylcarbonylamino-, Morpholin-4-ylcarbonylamino- oder eine C₁₋₄-Alkylsulfonylamino-Gruppe darstellt,
eine Hydroxygruppe,
eine C₁₋₄-Alkyloxygruppe,
eine durch 1 bis 3 Fluoratome substituierte Methoxy- oder Ethyloxy-Gruppe,
eine C₂₋₄-Alkyloxygruppe, die durch den Rest R⁵ substituiert ist, wobei R⁵ wie vorstehend erwähnt definiert ist,
eine C₃₋₇-Cycloalkyloxy- oder C₃₋₇-Cycloalkyl-C₁₋₄-alkyloxy-Gruppe,
eine Tetrahydrofuran-3-yloxy-, Tetrahydropyran-3-yloxy- oder Tetrahydropyran-4-yloxyGruppe,
eine Tetrahydrofuranyl-C₁₋₄-alkyloxy- oder Tetrahydropyranyl-C₁₋₄-alkyloxy-Gruppe,
eine C₁₋₄-Alkoxygruppe, die durch eine in 1-Stellung durch den Rest R⁶ substituierte Pyrrolidinyl-, Piperidinyl- oder Homopiperidinyl-Gruppe substituiert ist, wobei
   R⁶ ein Wasserstoffatom oder eine C₁₋₃-Alkylgruppe darstellt,
oder eine C₁₋₄-Alkoxygruppe, die durch eine in 4-Stellung durch den Rest R⁶ substituierte Morpholinylgruppe substituiert ist, wobei R⁶ wie vorstehend erwähnt definiert ist, und wobei die bei der Definition des Restes R^{c} vorstehend erwähnten Pyrrolidinyl-, Piperidinyl-, Piperazinyl- und Morpholinyl-Gruppen jeweils durch eine oder zwei C₁₋₃-Alkylgruppen substituiert sein können, und
wobei unter den bei der Definition der vorstehend genannten Reste erwähnten Arylgruppen jeweils eine Phenylgruppe zu verstehen ist, die durch R⁷ mono- oder disubstituiert ist, wobei die Substituenten gleich oder verschieden sein können und
   R⁷ ein Wasserstoffatom, ein Fluor-, Chlor-, Brom- oder lodatom oder eine C₁₋₃-Alkyl-, Hydroxy-, C₁₋₃-Alkyloxy-, Difluormethyl- Trifluormethyl-, Difluormethoxy-, Trifluormethoxy- oder Cyano-Gruppe darstellt, und
unter den bei der Definition der vorstehend genannten Reste erwähnten Heteroarylgruppen eine Pyridyl-, Pyridazinyl-, Pyrimidinyl- oder Pyrazinyl-Gruppe zu verstehen ist, wobei die vorstehend erwähnten Heteroarylgruppen jeweils durch den Rest R⁷ mono- oder disubstituiert sind, wobei die Substituenten gleich oder verschieden sein können und R⁷ wie vorstehend erwähnt definiert ist, und
soweit nichts anderes erwähnt wurde, die vorstehend erwähnten Alkylgruppen geradkettig oder verzweigt sein können.

Bevorzugte Verbindungen der obigen allgemeinen Formel I sind diejenigen, in denen
R^{a} eine Phenyl- oder 1-Phenylethyl-Gruppe, in denen der Phenylkern jeweils durch die Reste R¹ bis R³ substituiert ist, wobei
   R¹ ein Wasserstoff-, Fluor-, Chlor- oder Bromatom,
   eine Methyl-, Trifluormethyl- oder Ethinyl-Gruppe,
   eine Phenyloxy- oder Phenylmethoxy-Gruppe, wobei der Phenylteil der vorstehend erwähnten Gruppen gegebenenfalls durch ein Fluor- oder Chloratom substituiert ist, oder
   eine Pyridyloxy- oder Pyridinylmethoxy-Gruppe, wobei der Pyridinylteil der vorstehend erwähnten Gruppen gegebenenfalls durch eine Methyl- oder Trifluormethyl-Gruppe substituiert ist,
   R² ein Wasserstoff-, Fluor- oder Chloratom oder eine Methylgruppe und
   R³ ein Wasserstoffatom darstellen,
R^{b} eine Azetidin-1-yl-, Pyrrolidin-1-yl-, Piperidin-1-yl-, Homopiperidin-1-yl-, Morpholin-4-yl-, Homomorpholin-4-yl, Piperazin-1-yl-, 4-(C₁₋₃-Alkyl-carbonyl)-piperazin-1-yl-, 4-(C₁₋₃-Alkyl-sulfonyl)-piperazin-1-yl-, Homopiperazin-1-yl-, 4-(C₁₋₃-Alkyl-carbonyl)-homopiperazin-1-yl- oder 4-(C₁₋₃-Alkyl-sulfonyl)-homopiperazin-1-yl-Gruppe, die jeweils durch R⁴ mono- oder disubstituiert sein kann, wobei die Substituenten gleich oder verschieden sein können und
   R⁴ ein Fluoratom,
   eine C₁₋₃-Alkylgruppe,
   eine Amino-, C₁₋₃-Alkylamino-, Di-(C₁₋₃-alkyl)amino-, C₁₋₃-Alkyl-carbonylamino-, N-(C₁₋₃-Alkyl)-C₁₋₃-alkyl-carbonylamino-, C₁₋₃-Alkyl-sulfonylamino- oder N-(C₁₋₃-Alkyl)-C₁₋₃-alkyl-sulfonylamino-Gruppe,
   eine Amino-C₁₋₃-alkyl-, C₁₋₃-Alkylamino-C₁₋₃-alkyl-, Di-(C₁₋₃-alkyl)amino-C₁₋₃-alkyl-, C₁₋₃-Alkyl-carbonylamino-C₁₋₃-alkyl-, N-(C₁₋₃-Alkyl)-C₁₋₃-alkyl-carbonylamino-C₁₋₃-alkyl-, C₁₋₃-Alkyl-sulfonylamino-C₁₋₃-alkyl- oder N-(C₁₋₃-Alkyl)-C₁₋₃-alkyl-sulfonylamino-C₁₋₃-alkyl-Gruppe,
   eine Hydroxy-, C₁₋₃-Alkyloxy- oder C₁₋₃-Alkyl-carbonyloxy-Gruppe,
   eine Hydroxy-C₁₋₃-alkyl-, C₁₋₃-Alkyloxy-C₁₋₃-alkyl- oder C₁₋₃-Alkyl-carbonyloxy-C₁₋₃-alkyl-Gruppe,
   eine C₁₋₃-Alkyl-carbonyl-, Cyano-, C₁₋₄-Alkyl-oxycarbonyl-, Carboxy-, Aminocarbonyl-, C₁₋₃-Alkyl-aminocarbonyl- oder Di-(C₁₋₃-alkyl)amino-carbonyl-Gruppe,
   eine C₁₋₃-Alkylcarbonyl-C₁₋₃-alkyl-, Cyano-C₁₋₃-alkyl-, C₁₋₄-Alkyloxycarbonyl-C₁₋₃-alkyl-gruppe, Aminocarbonyl-C₁₋₃-alkyl-, C₁₋₃-Alkylaminocarbonyl-C₁₋₃-alkyl- oder Di-(C₁₋₃-alkyl)aminocarbonyl-C₁₋₃-alkyl-Gruppe,
   eine C₁₋₄-Alkylsulfanyl-, C₁₋₄-Alkylsulfinyl-, C₁₋₄-Alkylsulfonyl-, Aminosulfonyl-, C₁₋₃-Alkyl-aminosulfonyl- oder Di-(C₁₋₃-alkyl)amino-sulfonyl-Gruppe,
   eine C₁₋₄-Alkylsulfanyl-C₁₋₃-alkyl-, C₁₋₄-Alkylsulfinyl-C₁₋₃-alkyl-, C₁₋₄-Alkylsulfonyl-, C₁₋₃-alkyl-, Aminosulfonyl-C₁₋₃-alkyl-, C₁₋₃-Alkylaminosulfonyl-C₁₋₃-alkyl- oder Di-(C₁₋₃-alkyl)amino-sulfonyl-C₁₋₃-alkylGruppe
   darstellt, und wobei die vorstehend erwähnten Heterocylen zusätzlich durch eine Oxo-Gruppe substituiert sein können,
R^{c} ein Wasserstoffatom,
eine Hydroxygruppe,
eine C₁₋₃-Alkyloxygruppe,
eine Methoxygruppe, die durch ein bis drei Fluoratome substituiert ist,
eine Ethyloxygruppe, die in 2-Stellung durch einen Rest R⁵ substituiert ist, wobei
   R⁵ eine Hydroxy-, C₁₋₃-Alkyloxy-, Amino-, C₁₋₃-Alkylamino-, Di-(C₁₋₃-alkyl)amino-, Bis-(2-methoxyethyl)-amino-, Pyrrolidin-1-yl-, Piperidin-1-yl- , Morpholin-4-yl-, Homomorpholin-4-yl-, Piperazin-1-yl- oder eine 4-C₁₋₃-Alkyl- piperazin-1-yl-Gruppe darstellt,
eine Propyloxygruppe, die in 3-Stellung durch den Rest R⁵ substituiert ist, wobei R⁵ wie vorstehend erwähnt definiert ist, oder
eine Butyloxygruppe, die in 4-Stellung durch einen Rest R⁵ substituiert ist, wobei R⁵ wie vorstehend erwähnt definiert ist, und
wobei, soweit nichts anderes erwähnt wurde, die vorstehend erwähnten Alkylgruppen geradkettig oder verzweigt sein können,
deren Tautomere, deren Stereoisomere, deren Gemische und deren Salze.

Besonders bevorzugte Verbindungen der obigen allgemeinen Formel I sind diejenigen, in denen
R^{a} eine 1-Phenylethyl-, 3-Ethinylphenyl-, 3-Brom-2-fluor-phenyl-, 3-Brom-4-fluorphenyl-, 3-Chlor-2-fluor-phenyl- oder 3-Chlor-4-fluor-phenyl-Gruppe,
eine 3-Chlor-4-benzyloxy-phenyl-, 3-Chlor-4-[(3-fluor-benzyl)oxy]-phenyl-, 4-(Pyridin-3-yloxy)-phenyl-, 4-[(6-Methyl-pyridin-3-yl)oxy]-phenyl-, 3-Methyl-4-(pyridin-3-yloxy)-phenyl-, 3-Methyl-4-[(6-methyl-pyridin-3-yl)oxy]-phenyl-, 3-Chlor-4-(pyridin-3-yloxy)-phenyl- oder 3-Chlor-4-[(6-methyl-pyridin-3-yl)oxy]-phenyl-Gruppe,
R^{b} eine Azetidin-1-yl-, Pyrrolidin-1-yl-, Piperidin-1-yl-, Morpholin-4-yl-, Piperazin-1-yl-, 4-(C₁₋₃-Alkyl-carbonyl)-piperazin-1-yl- oder 4-(C₁₋₃-Alkyl-sulfonyl)-piperazin-1-yl-Gruppe, die jeweils durch R⁴ mono- oder disubstituiert sein kann, wobei die Substituenten gleich oder verschieden sein können und
   R⁴ ein Fluoratom,
   eine C₁₋₃-Alkylgruppe,
   eine Amino-, C₁₋₃-Alkylamino-, Di-(C₁₋₃-alkyl)amino-, C₁₋₃-Alkyl-carbonylamino-, N-(C₁₋₃-Alkyl)-C₁₋₃-alkyl-carbonylamino-, C₁₋₃-Alkyl-sulfonylamino- oder N-(C₁₋₃-Alkyl)-C₁₋₃-alkyl-sulfonylamino-Gruppe,
   eine Amino-C₁₋₃-alkyl-, C₁₋₃-Alkylamino-C₁₋₃-alkyl-, Di-(C₁₋₃-alkyl)amino-C₁₋₃-alkyl-, C₁₋₃-Alkyl-carbonylamino-C₁₋₃-alkyl-, N-(C₁₋₃-Alkyl)-C₁₋₃-alkyl-carbonylamino-C₁₋₃-alkyl-, C₁₋₃-Alkyl-sulfonylamino-C₁₋₃-alkyl- oder N-(C₁-3-Alkyl)-C₁₋₃-alkyl-sulfonylamino-C₁₋₃-alkyl-Gruppe,
   eine Hydroxy-, C₁₋₃-Alkyloxy- oder C₁₋₃-Alkyl-carbonyloxy-Gruppe,
   eine Hydroxy-C₁₋₃-alkyl-, C₁₋₃-Alkyloxy-C₁₋₃-alkyl- oder C₁₋₃-Alkyl-carbonyloxy-C₁₋₃-alkyl-Gruppe,
   eine C₁₋₃-Alkyl-carbonyl-, Cyano-, C₁₋₄-Alkyl-oxycarbonyl-, Carboxy-, Aminocarbonyl-, C₁₋₃-Alkyl-aminocarbonyl- oder Di-(C₁₋₃-alkyl)amino-carbonyl-Gruppe,
   eine C₁₋₃-Alkylcarbonyl-C₁₋₃-alkyl-, Cyano-C₁₋₃-alkyl-, C₁₋₄-Alkyloxycarbonyl-C₁₋₃-alkyl-gruppe, Aminocarbonyl-C₁₋₃-alkyl-, C₁₋₃-Alkylaminocarbonyl-C₁₋₃-alkyl- oder Di-(C₁₋₃-alkyl)aminocarbonyl-C₁₋₃-alkyl-Gruppe,
   eine C₁₋₄-Alkylsulfanyl-, C₁₋₄-Alkylsulfinyl-, C₁₋₄-Alkylsulfonyl-, Aminosulfonyl-, C₁₋₃-Alkyl-aminosulfonyl- oder Di-(C₁₋₃-alkyl)amino-sulfonyl-Gruppe,
   eine C₁₋₄-Alkylsulfanyl-C₁₋₃-alkyl-, C₁₋₄-Alkylsulfinyl-C₁₋₃-alkyl-, C₁₋₄-Alkylsulfonyl-C₁₋₃-alkyl-, Aminosulfonyl-C₁₋₃-alkyl-, C₁₋₃-Alkylaminosulfonyl-C₁₋₃-alkyl- oder Di-(C₁₋₃-alkyl)amino-sulfonyl-C₁₋₃-alkyl-Gruppe, darstellt, und wobei die vorstehend erwähnten Heterocylen zusätzlich durch eine Oxo-Gruppe substituiert sein können,
R^{c} ein Wasserstoffatom,
eine Methoxy- oder Ethyloxy-Gruppe,
eine Ethyloxygruppe, die in 2-Stellung durch den Rest R⁵ substituiert ist, wobei
   R⁵ eine Hydroxy-, Methoxy-, Ethoxy-, Amino-, Dimethylamino-, Diethylamino-, Bis-(2-methoxyethyl)-amino-, Pyrrolidin-1-yl-, Piperidin-1-yl-, Morpholin-4-yl-, Homomorpholin-4-yl-, Piperazin-1-yl-, 4-Methylpiperazin-1-yl- oder 4-Ethylpiperazin-1-yl-Gruppe darstellt,
eine Propyloxygruppe, die in 3-Stellung durch den Rest R⁵ substituiert ist, wobei R⁵ wie vorstehend erwähnt definiert ist, oder
eine Butyloxygruppe, die in 4-Stellung durch den Rest R⁵ substituiert ist, wobei R⁵ wie vorstehend erwähnt definiert ist, und
wobei, soweit nichts anderes erwähnt wurde, die vorstehend erwähnten Alkylgruppen geradkettig oder verzweigt sein können,
deren Tautomere, deren Stereoisomere, deren Gemische und deren Salze.

Ganz besonders bevorzugte Verbindungen der allgemeinen Formel I sind diejenigen, in denen
R^{a} eine 1-Phenylethyl-, 3-Ethinylphenyl-, 3-Brom-2-fluor-phenyl-, 3-Brom-4-fluor-phenyl-, 3-Chlor-2-fluor-phenyl- oder 3-Chlor-4-fluor-phenyl-Gruppe,
R^{b} eine Azetidin-1-yl-, Pyrrolidin-1-yl-, Piperidin-1-yl-, Morpholin-4-yl-, Piperazin-1-yl-, 4-(C₁₋₃-Alkyl-carbonyl)-piperazin-1-yl- oder 4-(C₁₋₃-Alkyl-sulfonyl)-piperazin-1-yl-Gruppe, die jeweils durch R⁴ mono- oder disubstituiert sein kann, wobei die Substituenten gleich oder verschieden sein können und
   R⁴ ein Fluoratom,
   eine C₁₋₃-Alkylgruppe,
   eine Amino-, C₁₋₂-Alkylamino-, Di-(C₁₋₂-alkyl)amino-, C₁₋₂-Alkyl-carbonylamino-, N-(C₁₋₂-Alkyl)-C₁₋₂-alkyl-carbonylamino-, C₁₋₂-Alkyl-sulfonylamino- oder N-(C₁₋₂-Alkyl)-C₁₋₂-alkyl-sulfonylamino-Gruppe,
   eine Amino-C₁₋₂-alkyl-, C₁₋₂-Alkylamino-C₁₋₂-alkyl-, Di-(C₁₋₂-alkyl)amino-C₁₋₂-alkyl-, C₁₋₂-Alkyl-carbonylamino-C₁₋₂-alkyl-, N-(C₁₋₂-Alkyl)-C₁₋₂-alkyl-carbonylamino-C₁₋₂-alkyl-, C₁₋₂-Alkyl-sulfonylamino-C₁₋₂-alkyl- oder N-(C₁₋₂ - Alkyl)-C₁₋₂-alkyl-sulfonylamino-C₁₋₂-alkyl-Gruppe,
   eine Hydroxy-, C₁₋₂-Alkyloxy- oder C₁₋₂-Alkyl-carbonyloxy-Gruppe,
   eine Hydroxy-C₁₋₂-alkyl-, C₁₋₂-Alkyloxy-C₂₋₄-alkyl- oder C₁₋₂-Alkyl-carbonyloxy-C₁₋₂-alkyl-Gruppe,
   eine C₁₋₂-Alkyl-carbonyl-, Cyano-, C₁₋₂-Alkyl-oxycarbonyl-, Carboxy-, Aminocarbonyl-, C₁₋₂-Alkyl-aminocarbonyl- oder Di-(C₁₋₂-alkyl)amino-carbonyl-Gruppe,
   eine C₁₋₂-Alkylcarbonyl-C₁₋₂-alkyl-, Cyano-C₁₋₂-alkyl-, C₁₋₂-Alkyloxycarbonyl-C₁₋₂-alkyl-gruppe, Aminocarbonyl-C₁₋₂-alkyl-, C₁₋₂-Alkylaminocarbonyl-C₁₋₂-alkyl- oder Di-(C₁₋₂-alkyl)aminocarbonyl-C₁₋₂-alkyl-Gruppe,
   eine C₁₋₂-Alkylsulfanyl-, C₁₋₂-Alkylsulfinyl- oder C₁₋₂-Alkylsulfonyl-Gruppe,
   eine C₁₋₂-Alkylsulfanyl-C₁₋₂-alkyl-, C₁₋₂-Alkylsulfinyl-C₁₋₂-alkyl- oder C₁₋₂-Alkylsulfonyl-C₁₋₂-alkyl-Gruppe,
   darstellt, und wobei die vorstehend erwähnten Heterocylen zusätzlich durch eine Oxo-Gruppe substituiert sein können,
R^{c} ein Wasserstoffatom,
eine Methoxy-, Ethyloxy- oder 2-(Methoxy)-ethyloxy-Gruppe,
eine 2-(Morpholin-4-yl)ethyloxy-, 3-(Morpholin-4-yl)propyloxy- oder 4-(Morpholin-4-yl)butyloxy-Gruppe,
bedeuten,
deren Tautomere, deren Stereoisomere, deren Gemische und deren Salze.

Insbesondere bevorzugte Verbindungen der allgemeinen Formel I sind diejenigen, in denen
R^{a} eine 1-Phenylethyl, 3-Ethinylphenyl-, 3-Chlor-2-fluor-phenyl- oder 3-Chlor-4-fluorphenyl-Gruppe,
R^{b} eine Azetidin-1-yl-, Pyrrolidin-1-yl-, Piperidin-1-yl-, Morpholin-4-yl-, Piperazin-1-yl-, 3-Oxo-piperazin-1-yl-, 4-Acetyl-piperazin-1-yl- oder 4-Methylsulfonyl-piperazin-1-yl-Gruppe, die jeweils durch R⁴ mono- oder disubstituiert sein kann, wobei die Substituenten gleich oder verschieden sein können und
   R⁴ eine Methyl-, Hydroxy-, Methoxy-, Cyano-, Aminocarbonyl-, Methylaminocarbonyl- oder Dimethylamino-carbonyl-Gruppe darstellt, und
R^{c} ein Wasserstoffatom,
eine Methoxy-, Ethyloxy- oder 2-(Methoxy)-ethyloxy-Gruppe,
bedeuten,
deren Tautomere, deren Stereoisomere, deren Gemische und deren Salze.

Unter den vorstehend beschriebenen bicyclischen Heterocyclen der allgemeinen Formel I sowie den jeweils als bevorzugt, besonders bevorzugt, ganz besonders bevorzugt und insbesondere bevorzugt gekennzeichneten Untergruppen sind jeweils diejenigen Verbindungen besonders hervorzuheben, in denen
(a) R^{a} eine 3-Ethinylphenyl-Gruppe darstellt,
(b) R^{a} eine 3-Chlor-2-fluor-phenyl-Gruppe darstellt,
(c) R^{a} eine 3-Chlor-4-fluor-phenyl-Gruppe darstellt oder
(d) R^{a} eine 1-Phenylethyl-Gruppe darstellt, und
(e) R^{c} eine Methoxy-Gruppe darstellt,
(f) R^{c} eine Ethyloxy-Gruppe darstellt oder
(g) R^{c} eine 2-Methoxy-ethyloxy-Gruppe darstellt,
wobei für R^{a} insbesondere die 3-Chlor-2-fluor-phenyl-Gruppe und für R^{c} die Methoxy-Gruppe hervorzuheben ist, und
wobei R^{b} jeweils wie vorstehend erwähnt definiert ist.

Beispielsweise seien folgende besonders bevorzugte Verbindungen der allgemeinen Formel I erwähnt:
(a) 4-[(3-Chlor-2-fluor-phenyl)amino]-6-[cis-4-(morpholin-4-yl)-cyclohexyloxy]-7-methoxy-chinazolin,
(b) 4-[(3-Chlor-2-fluor-phenyl)amino]-6-[trans-4-(morpholin-4-yl)-cyclohexyloxy]-7-methoxy-chinazolin,
(c) 4-[(3-Chlor-2-fluor-phenyl)amino]-6-[(R)-cis-4-(3-hydroxy-pyrrolidin-1-yl)-cyclohexyloxy]-7-methoxy-chinazolin,
(d) 4-[(3-Chlor-2-fluor-phenyl)amino]-6-[(R)-trans-4-(3-hydroxy-pyrrolidin-1-yl)-cyclohexyloxy]-7-methoxy-chinazolin,
(e) 4-[(3-Chlor-2-fluor-phenyl)amino]-6-[(S)-cis-4-(3-hydroxy-pyrrolidin-1-yl)-cyclohexyloxy]-7-methoxy-chinazolin,
(f) 4-[(3-Chlor-2-fluor-phenyl)amino]-6-[(S)-trans-4-(3-hydroxy-pyrrolidin-1-yl)-cyclohexyloxy]-7-methoxy-chinazolin,
(g) 4-[(3-Chlor-2-fluor-phenyl)amino]-6-[cis-4-(3-oxo-piperazin-1-yl)-cyclohexyloxy]-7- methoxy-chinazolin,
(h) 4-[(3-Chlor-2-fluor-phenyl)amino]-6-[trans-4-(3-oxo-piperazin-1-yl)-cyclohexyloxy]-7-methoxy-chinazolin,
(i) 4-[(3-Chlor-2-fluor-phenyl)amino]-6-{(S)-cis-4-[2-(N,N-dimethylaminocarbonyl)-pyrrolidin-1-yl]-cyclohexyloxy]-7-methoxy-chinazolin,
(j) 4-[(3-Chlor-2-fluor-phenyl)amino]-6-{(S)-trans-4-[2-(N,N-dimethylaminocarbonyl)- pyrrolidin-1-yl]-cyclohexyloxy]-7-methoxy-chinazolin,
(k) 4-[(3-Chlor-2-fluor-phenyl)amino]-6-{(S)-cis-4-[2-(aminocarbonyl)-pyrrolidin-1-yl]- cyclohexyloxy]-7-methoxy-chinazolin und
(l) 4-[(3-Chlor-2-fluor-phenyl)amino]-6-{(S)-trans-4-[2-(aminocarbonyl)-pyrrolidin-1- yl]-cyclohexyloxy]-7-methoxy-chinazolin,
sowie deren Salze.

Die Verbindungen der allgemeinen Formel I lassen sich beispielsweise nach folgenden Verfahren herstellen:
a) Umsetzung einer Verbindung der allgemeinen Formel 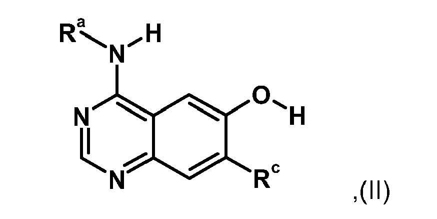 in der
   R^{a} und R^{c} wie eingangs erwähnt definiert sind, mit einer Verbindung der allgemeinen Formel 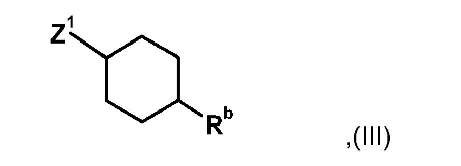 in der
      R^{b} wie eingangs erwähnt definiert ist und Z¹ eine Austrittsgruppe wie ein Halogenatom, z.B. ein Chlor- oder Bromatom, eine Sulfonyloxygruppe wie eine Methansulfonyloxy- oder p-Toluolsulfonyloxygruppe oder eine Hydroxygruppe darstellt.
   Mit einer Verbindung der allgemeinen Formel (III), in der Z¹ ein Halogenatom oder eine Sulfonyloxygruppe darstellt, erfolgt die Umsetzung zweckmäßigerweise in einem Lösungsmittel wie Ethanol, Isopropanol, Acetonitril, Toluol, Tetrahydrofuran, Dioxan, Dimethylformamid, Dimethylsulfoxid oder N-Methylpyrrolidinon, vorzugsweise in Gegenwart einer Base wie Kaliumcarbonat, Kalium-tert-butylat, Natriumhydrid oder N-Ethyl-diisopropylamin, bei Temperaturen im Bereich von 20°C bis 160°C, beispielsweise bei Temperaturen im Bereich von 80°C bis 140°C.
   Mit einer Verbindung der allgemeinen Formel III, in der Z¹ eine Hydroxygruppe darstellt, wird die Umsetzung in Gegenwart eines wasserentziehenden Mittels, vorzugsweise in Gegenwart eines Phosphins und eines Azodicarbonsäurederivates wie z.B. Triphenylphosphin/Azodicarbonsäurediethylester, zweckmäßigerweise in einem Lösungsmittel wie Methylenchlorid, Acetonitril, Tetrahydrofuran, Dioxan, Toluol oder Ethylenglycoldiethylether bei Temperaturen zwischen -50 und 150°C, vorzugsweise jedoch bei Temperaturen zwischen -20 und 80°C, durchgeführt.
b) Umsetzung einer Verbindung der allgemeinen Formel 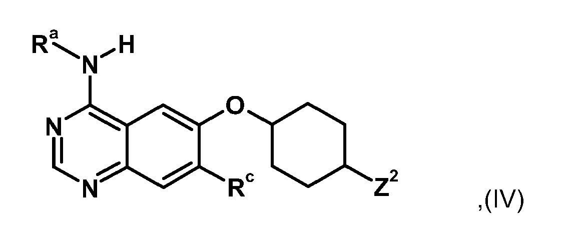 in der
   R^{a} und R^{c} wie eingangs erwähnt definiert sind, und Z² eine Austrittsgruppe wie ein Halogenatom, z.B. ein Chlor- oder Bromatom oder eine Sulfonyloxygruppe wie eine Methansulfonyloxy- oder p-Toluolsulfonyloxygruppe darstellt, mit einer Verbindung der allgemeinen Formel

      H-R^{b} ,(V)

      in der
      R^{b} wie eingangs erwähnt definiert ist.
   Die Reaktion wird vorzugsweise in Gegenwart einer organischen oder anorganischen Base wie Kaliumcarbonat oder N-Ethyl-diisopropylamin beispielsweise in einem Lösemittel wie bei Ethanol, Isopropanol, Acetonitril, Toluol, Tetrahydrofuran, Dioxan, Dimethylformamid, Dimethylsulfoxid oder N-Methylpyrrolidinon bei Temperaturen im Bereich von 0°C und 150°C durchgeführt.
c) Umsetzung einer Verbindung der allgemeinen Formel 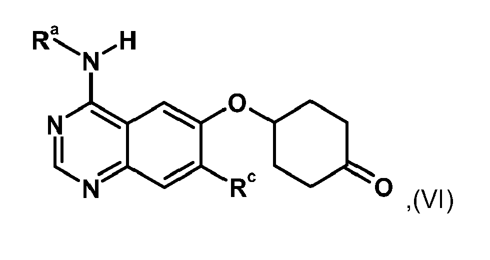 in der
   R^{a} und R^{c} wie eingangs erwähnt definiert sind, mit einer Verbindung der allgemeinen Formel

      H-R^{b} ,(VII)

      in der
      R^{b} wie eingangs erwähnt definiert ist, in Gegenwart eines Reduktionsmittel.
   Die reduktive Aminierung wird beispielsweise in einem Lösemittel wie Dichlormethan, 1,2-Dichlorethan, Methanol, Ethanol, Tetrahydrofuran oder Dioxan in Gegenwart eines Reduktionsmittels wie Natrium-triacetoxyborhydrid oder Natriumcyanborhydrid, gegebenenfalls in Gegenwart von Essigsäure bei Temperaturen zwischen 0°C und 80°C durchgeführt. Die reduktive Aminierung kann auch mit Wasserstoff in Gegenwart eines Katalysators wie Palladium auf Aktivkohle oder Platinoxid erfolgen. Eine weitere Möglichkeit besteht darin, aus dem Keton der allgemeinen Formel VI und dem Amin der allgemeinen Formel VII unter Wasserabspaltung, beispielsweise mit Titan(IV)-isopropylat, das Enamin zu bilden und dieses anschließend zu reduzieren, beispielsweise mit Natriumborhydrid oder Wasserstoff/Palladium auf Aktivkohle.
d) Umsetzung einer Verbindung der allgemeinen Formel (VIII) 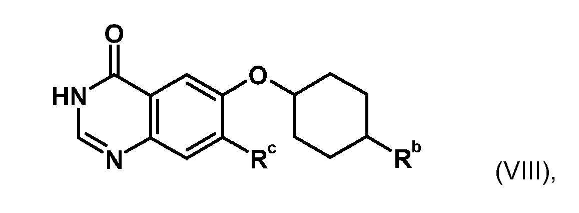 in der R^{b} und R^{c} wie eingangs erwähnt definiert sind, mit einem Halogenierungsmittel, beispielsweise einem Säurehalogenid wie Thionylchlorid, Thionylbromid, Phosphortrichlorid, Phosphorpentachlorid oder Phosphoroxychlorid zu einer Zwischenverbindung der allgemeinen Formel (IX), 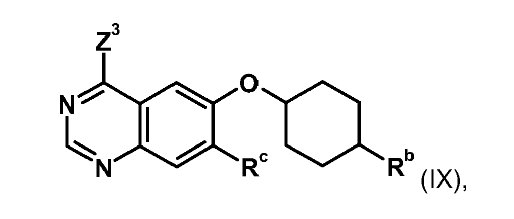 in der R^{b} und R^{c} wie eingangs erwähnt definiert sind und Z³ ein Halogenatom wie ein Chlor- oder Bromatom darstellt,
   und anschließender Umsetzung mit einer Verbindung der allgemeinen Formel (X),
   R^{a}-NH₂ (X), in der R^{a}wie eingangs erwähnt definiert ist, oder dessen Salzen.
   Die Umsetzung mit dem Halogenierungsmittel wird gegebenenfalls in einem Lösungsmittel wie Methylenchlorid, Chloroform, Acetonitril oder Toluol und gegebenfalls in Gegenwart einer Base wie N,N-Diethylanilin oder N-Ethyl-diisopropylamin bei Temperaturen im Bereich von 20°C bis 160°C, vorzugsweise von 40°C bis 120°C durchgeführt. Vorzugsweise wird die Reaktion jedoch mit Thionylchlorid und katalytischen Mengen an Dimethylformamid bei der Siedetemperatur des Reaktionsgemisches oder aber mit Phosphoroxychlorid in Acetonitril in Gegenwart von Triethylamin bei der Siedetemperatur des Reaktionsgemisches durchgeführt.
   Die Umsetzung der Verbindung der allgemeinen Formel (IX) mit der Verbindung der allgemeinen Formel (X) oder deren Salzen erfolgt zweckmäßigerweise in einem Lösungsmittel wie Ethanol, Isopropanol, Acetonitril, Dioxan oder Dimethylformamid, gegebenfalls in Gegenwart einer Base wie Kaliumcarbonat oder N-Ethyl-diisopropylamin, bei Temperaturen im Bereich von 20°C und 160°C, vorzugsweise von 60°C bis 120°C. Vorzugsweise wird die Reaktion jedoch in Isopropanol bei der Siedetemperatur des Reaktionsgemisches durchgeführt.
e) Zur Herstellung von Verbindungen der allgemeinen Formel I, in der R^{c} eine der eingangs erwähnten, gegebenenfalls substituierten Alkyloxygruppen darstellt:
   Umsetzung einer Verbindung der allgemeinen Formel 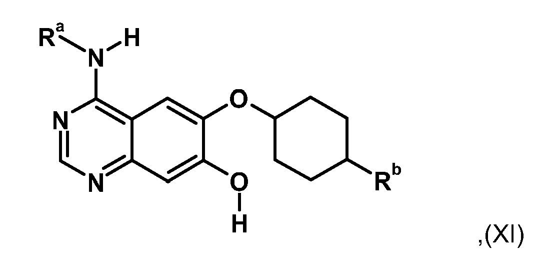 in der R^{a} und R^{b} wie eingangs erwähnt definiert, sind mit einer mit einer Verbindung der allgemeinen Formel

      Z⁴-R^{c'} ,(XII)

      in der R^{c'} eine C₁₋₄-Alkylgruppe, eine durch 1 bis 3 Fluoratome substituierte Methyl- oder Ethylgruppe, eine C₃₋₇-Cycloalkyl- oder C₃₋₇-Cycloalkyl-C₁₋₄-alkylgruppe, eine Tetrahydrofuran-3-yl-, Tetrahydropyran-3-yl- oder Tetrahydropyran-4-ylgruppe, eine Tetrahydrofuranyl-C₁₋₄-alkyl- oder Tetrahydropyranyl-C₁₋₄-alkylgruppe, eine durch R⁷ substituierte C₂₋₄-Alkylgruppe, wobei R⁷ wie eingangs erwähnt definiert ist, eine C₁₋₄-Alkylgruppe, die durch eine in 1-Stellung durch den Rest R⁸ substituierte Pyrrolidinyl-, Piperidinyl- oder Homopiperidinylgruppe substituiert ist, oder eine C₁₋₄-Alkylgruppe, die durch eine in 4-Stellung durch den Rest R⁸ substituierte Morpholinylgruppe substituiert ist, wobei R⁸ jeweils wie eingangs erwähnt definiert ist, darstellt und
      Z⁴ eine Austrittsgruppe wie ein Halogenatom, eine Alkylsulfonyloxy-, Arylsulfonyloxy- oder eine Hydroxygruppe darstellt.
   Handelt es sich bei der Austrittsgruppe um ein Halogenatom wie ein Chlor-, Brom- oder lodatom oder um eine Alkylsulfonyloxy- oder Arylsulfonyloxygruppe wie die die Methansulfonyloxy oder p-Toluolsulfonyloxygruppe, wird die Reaktion vorzugsweise in Gegenwart einer organischen oder anorganischen Base wie Kaliumcarbonat, Natriumhydrid oder N-Ethyl-diisopropylamin durchgeführt. Handelt es sich bei der Austrittsgruppe um eine Hydroxygruppe, so wird die Umsetzung in Gegenwart eines wasserentziehenden Mittels, vorzugsweise in Gegenwart eines Phosphins und eines Azodicarbonsäurederivates wie z.B. Triphenylphosphin/Azodicarbonsäurediethylester durchgeführt.
f) Zur Herstellung von Verbindungen der allgemeinen Formel I, in der R^{c} eine der eingangs erwähnten Alkyloxygruppen darstellt, die durch eine gegebenenfalls substituierte Amino-, Alkylamino- oder Dialkylaminogruppe oder durch eine gegebenenfalls substituierte, über ein Iminostickstoffatom gebundene heterocyclischen Gruppe substituiert ist: Umsetzung einer Verbindung der allgemeinen Formel 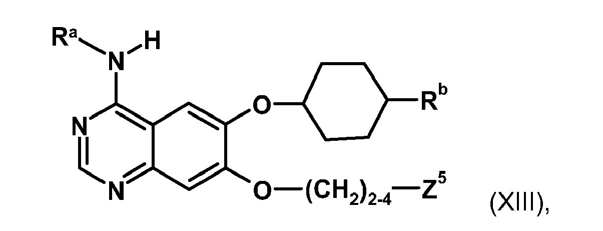 in der R^{a} und R^{b} wie eingangs erwähnt definiert sind und Z⁵ eine Austrittsgruppe wie ein Halogenatom, z.B. ein Chlor- oder Bromatom oder eine Sulfonyloxygruppe wie eine Methansulfonyloxy- oder p-Toluolsulfonyloxygruppe darstellt, mit
   Ammoniak, einem entsprechenden, gegebenenfalls substituierten Alkylamin, Dialkylamin oder einer Iminoverbindung oder deren geeigneten Salzen oder Derivaten, wie beispielsweise Morpholin.
g) Zur Herstellung von Verbindungen der allgemeinen Formel 1, in der R^{b} eine oder mehrere Hydroxygruppen enthält:
   Abspaltung von Schutzresten von einer Verbindung der allgemeinen Formel 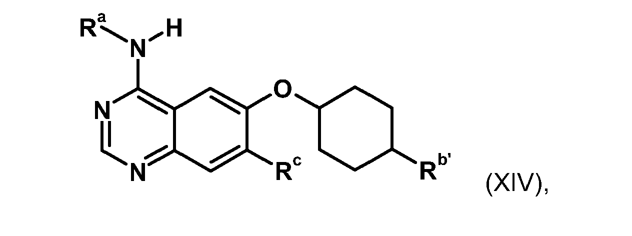 in der R^{a} und R^{c} wie eingangs erwähnt definiert sind und R^{b'} eine oder mehrere in Hydroxygruppen überführbare Gruppen enthält, beispielsweise eine gegebenenfalls substituierte Benzyloxygruppe, eine Silyloxy-, Acetyloxy-, Benzoyloxy-, Methoxy-, Ethoxy-, tert-Butoxy- oder Trityloxygruppe.
   Die Abspaltung der Schutzreste erfolgt beispielsweise hydrolytisch in einem wässrigen Lösungsmittel, z.B. in Wasser, Isopropanol/Wasser, Essigsäure/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Natriumhydroxid oder Kaliumhydroxid oder aprotisch, z.B. in Gegenwart von Jodtrimethylsilan, bei Temperaturen zwischen 0 und 120°C, vorzugsweise bei Temperaturen zwischen 10 und 100°C.
   Die Abspaltung eines Benzyl- oder Methoxybenzylrestes erfolgt beispielsweise hydrogenolytisch, z.B. mit Wasserstoff in Gegenwart eines Katalysators wie Palladium/Kohle in einem geeigneten Lösungsmittel wie Methanol, Ethanol, Essigsäureethylester oder Eisessig gegebenenfalls unter Zusatz einer Säure wie Salzsäure bei Temperaturen zwischen 0 und 100°C, vorzugsweise jedoch bei Raumtemperaturen zwischen 20 und 60°C, und bei einem Wasserstoffdruck von 1 bis 7 bar, vorzugsweise jedoch von 3 bis 5 bar. Die Abspaltung eines 2,4-Dimethoxybenzylrestes erfolgt jedoch vorzugsweise in Trifluoressigsäure in Gegenwart von Anisol.
   Die Abspaltung eines tert.-Butyl- oder Benzylrestes erfolgt beispielsweise durch Behandlung mit einer Säure wie Trifluoressigsäure, Salzsäure oder Bromwasserstoffsäure oder durch Behandlung mit Jodtrimethylsilan gegebenenfalls unter Verwendung eines Lösungsmittels wie Methylenchlorid, Dioxan, Methanol oder Diethylether.
   Die Spaltung einer Silyloxygruppe, beispielsweise einer tert.-Butyl-dimethylsilylgruppe, erfolgt beispielsweise durch Behandlung mit Fluoriden wie Tetrabutylammoniumfluorid gegebenenfalls unter Verwendung eines Lösungsmittels wie Tetrahydrofuran oder Dioxan.
h) Zur Herstellung von Verbindungen der allgemeinen Formel I, in der R^{b} eine -NH-Gruppe enthält:
   Abspaltung eines Schutzrestes von einer Verbindung der allgemeinen Formel 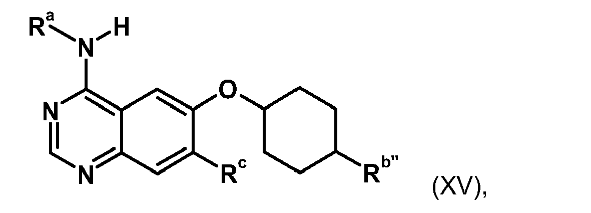
      in der R^{a} und R^{c} wie eingangs erwähnt definiert sind und R^{b"} mit der Maßgabe die eingangs für R^{b} erwähnten Bedeutungen besitzt, daß R^{b"} ein geschütztes Stickstoffatom enthält.

Übliche Schutzreste für eine Amino-, Alkylamino- oder Iminogruppe sind beispielsweise die Formyl-, Acetyl-, Trifluoracetyl-, Ethoxycarbonyl-, tert.-Butoxycarbonyl-, Benzyloxycarbonyl-, Benzyl-, Methoxybenzyl- oder 2,4-Dimethoxybenzylgruppe , wobei für die Aminogruppe zusätzlich die Phthalylgruppe in Betracht kommt.

Die Abspaltung des Schutzrestes erfolgt beispielsweise hydrolytisch in einem wässrigen Lösungsmittel, z.B. in Wasser, Isopropanol/Wasser, Essigsäure/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Natriumhydroxid oder Kaliumhydroxid oder aprotisch, z.B. in Gegenwart von Jodtrimethylsilan, bei Temperaturen zwischen 0 und 120°C, vorzugsweise bei Temperaturen zwischen 10 und 100°C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem geeigneten Lösungsmittel wie Methanol, Ethanol, Essigsäureethylester oder Eisessig gegebenenfalls unter Zusatz einer Säure wie Salzsäure bei Temperaturen zwischen 0 und 100°C, vorzugsweise jedoch bei Raumtemperaturen zwischen 20 und 60°C, und bei einem Wasserstoffdruck von 1 bis 7 bar, vorzugsweise jedoch von 3 bis 5 bar. Die Abspaltung eines 2,4-Dimethoxybenzylrestes erfolgt jedoch vorzugsweise in Trifluoressigsäure in Gegenwart von Anisol.

Die Abspaltung eines tert.-Butyl- oder tert.-Butyloxycarbonylrestes erfolgt vorzugsweise durch Behandlung mit einer Säure wie Trifluoressigsäure oder Salzsäure oder durch Behandlung mit Jodtrimethylsilan gegebenenfalls unter Verwendung eines Lösungsmittels wie Methylenchlorid, Dioxan, Methanol oder Diethylether.

Die Abspaltung eines Trifluoracetylrestes erfolgt vorzugsweise durch Behandlung mit einer Säure wie Salzsäure gegebenenfalls in Gegenwart eines Lösungsmittels wie Essigsäure bei Temperaturen zwischen 50 und 120°C oder durch Behandlung mit Natronlauge gegebenenfalls in Gegenwart eines Lösungsmittels wie Tetrahydrofuran bei Temperaturen zwischen 0 und 50°C.

Die Abspaltung eines Phthalylrestes erfolgt vorzugsweise in Gegenwart von Hydrazin oder eines primären Amins wie Methylamin, Ethylamin, n-Butylamin oder Ethanolamin in einem Lösungsmittel wie Methanol, Ethanol, Isopropanol, Toluol/Wasser oder Dioxan bei Temperaturen zwischen 20 und 50°C.

Erhält man erfindungsgemäß eine Verbindung der allgemeinen Formel I, die eine Amino-, Alkylamino- oder Iminogruppe enthält, so kann diese mittels Acylierung oder Sulfonylierung in eine entsprechende Acyl- oder Sulfonylverbindung der allgemeinen Formel I übergeführt werden, wobei als Acylierungsmittel beispielsweise Carbonsäurehalogenide, Carbonsäureanhydride und Carbonsäuren mit Aktivierungsmitteln wie N,N'-Carbonyldümidazol, N,N'-Dicyclohexylcarbodümid oder O-(Benzotriazol-1-yl)-N,N,N'N'-tetramethyluronium-tetrafluoroborat und als Sulfonylierungsmittel Sulfonylhalogenide in Frage kommen, und/oder
eine Verbindung der allgemeinen Formel I, die eine Amino-, Alkylamino- oder Iminogruppe enthält, so kann diese mittels Alkylierung oder reduktiver Alkylierung in eine entsprechende Alkylverbindung der allgemeinen Formel I übergeführt werden und/oder
eine Verbindung der allgemeinen Formel I, die eine Alkoxycarbonylgruppe enthält, so kann diese durch Esterspaltung in eine Carbonsäure übergeführt werden und/oder
eine Verbindung der allgemeinen Formel I, die eine Alkoxycarbonylgruppe enthält, so kann diese durch Umsetzung mit einem Amin in ein Carbonsäureamid-Derivat übergeführt werden und/oder
eine Verbindung der allgemeinen Formel I, die eine Carboxy-Gruppe enthält, so kann diese durch Umsetzung mit einem Amin in ein Carbonsäureamid-Derivat übergeführt werden.

Bei den vorstehend beschriebenen Umsetzungen können gegebenenfalls vorhandene reaktive Gruppen wie Hydroxy-, Amino-, Alkylamino- oder Iminogruppen während der Umsetzung durch übliche Schutzgruppen geschützt werden, welche nach der Umsetzung wieder abgespalten werden.

Beispielsweise kommen als Schutzrest für eine Hydroxygruppe die Trimethylsilyl-, Acetyl-, Trityl-, Benzyl- oder Tetrahydropyranylgruppe in Betracht.

Als Schutzreste für eine Amino-, Alkylamino- oder Iminogruppe kommen beispielsweise die Formyl-, Acetyl-, Trifluoracetyl-, Ethoxycarbonyl-, tert.-Butoxycarbonyl-, Benzyloxycarbonyl-, Benzyl-, Methoxybenzyl- oder 2,4-Dimethoxybenzylgruppe in Betracht.

Die gegebenenfalls anschließende Abspaltung eines verwendeten Schutzrestes erfolgt beispielsweise hydrolytisch in einem wässrigen Lösungsmittel, z.B. in Wasser, Isopropanol/Wasser, Essigsäure/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Natriumhydroxid oder Kaliumhydroxid oder aprotisch, z.B. in Gegenwart von Jodtrimethylsilan, bei Temperaturen zwischen 0 und 120°C, vorzugsweise bei Temperaturen zwischen 10 und 100°C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem geeigneten Lösungsmittel wie Methanol, Ethanol, Essigsäureethylester oder Eisessig gegebenenfalls unter Zusatz einer Säure wie Salzsäure bei Temperaturen zwischen 0 und 100°C, vorzugsweise jedoch bei Raumtemperaturen zwischen 20 und 60°C, und bei einem Wasserstoffdruck von 1 bis 7 bar, vorzugsweise jedoch von 3 bis 5 bar. Die Abspaltung eines 2,4-Dimethoxybenzylrestes erfolgt jedoch vorzugsweise in Trifluoressigsäure in Gegenwart von Anisol.

Die Abspaltung eines tert.-Butyl- oder tert.-Butyloxycarbonylrestes erfolgt vorzugsweise durch Behandlung mit einer Säure wie Trifluoressigsäure oder Salzsäure oder durch Behandlung mit Jodtrimethylsilan gegebenenfalls unter Verwendung eines Lösungsmittels wie Methylenchlorid, Dioxan, Methanol oder Diethylether.

Die Abspaltung eines Trifluoracetylrestes erfolgt vorzugsweise durch Behandlung mit einer Säure wie Salzsäure gegebenenfalls in Gegenwart eines Lösungsmittels wie Essigsäure bei Temperaturen zwischen 50 und 120°C oder durch Behandlung mit Natronlauge gegebenenfalls in Gegenwart eines Lösungsmittels wie Tetrahydrofuran oder Methanol bei Temperaturen zwischen 0 und 50°C.

Ferner können die erhaltenen Verbindungen der allgemeinen Formel I, wie bereits eingangs erwähnt wurde, in ihre Enantiomeren und/oder Diastereomeren aufgetrennt werden. So können beispielsweise cis-/trans-Gemische in ihre cis- und trans-Isomere, und Verbindungen mit mindestens einem optisch aktiven Kohlenstoffatom in ihre Enantiomeren aufgetrennt werden.

So lassen sich beispielsweise die erhaltenen cis-/trans-Gemische durch Chromatographie in ihre cis- und trans-Isomeren, die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971)) in ihre optischen Antipoden und Verbindungen der allgemeinen Formel I mit mindestens 2 asymmetrischen Kohlenstoffatomen auf Grund ihrer physikalisch-chemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-o-Tolylweinsäure, Äpfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise (+)-oder (-)-Menthyloxycarbonyl in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren oder Basen übergeführt werden. Als Säuren kommen hierfür beispielsweise Salzsäure, Bromwasserstoffsäure, Schwefelsäure, Methansulfonsäure, Phosphorsäure, Fumarsäure, Bernsteinsäure, Milchsäure, Zitronensäure, Weinsäure oder Maleinsäure in Betracht. Als Basen kommen hierfür beispielsweise Natronlauge, Kalilauge, Calciumhydroxid, Diethanolamin oder N-Methyl-D-glucamine in Betracht.

Unter dem Begriff "C₁₋₄-Alkyl" (auch soweit sie Bestandteil anderer Reste sind) werden verzweigte und unverzweigte Alkylgruppen mit 1 bis 4 Kohlenstoffatomen verstanden. Beispielsweise werden hierfür genannt: Methyl, Ethyl, *n*-Propyl, *iso-*Propyl, *n*-Butyl, *iso*-Butyl, *sec*-Butyl oder *tert*-Butyl,. Gegebenenfalls werden für die vorstehend genannten Gruppen auch die Abkürzungen Me, Et, *n*-Pr, *i*-Pr, *n*-Bu, *i*-Bu, *t*-Bu, etc. verwendet. Sofern nicht anders beschrieben, umfassen die Definitionen Propyl und Butyl, alle denkbaren isomeren Formen der jeweiligen Reste. So umfasst beispielsweise Propyl *n*-Propyl und *iso*-Propyl, Butyl umfasst *iso*-Butyl, *sec*-Butyl und *tert*-Butyl etc.

Unter dem Begriff "C₂₋₃-Alkenyl" (auch soweit sie Bestandteil anderer Reste sind) werden verzweigte und unverzweigte Alkenylgruppen mit 2 bis 3 Kohlenstoffatomen, soweit sie mindestens eine Doppelbindung aufweisen. Beispielsweise werden hierfür genannt: Ethenyl oder Vinyl,.

Unter dem Begriff "C₂₋₃-Alkinyl" (auch soweit sie Bestandteil anderer Reste sind) werden verzweigte und unverzweigte Alkinylgruppen mit 2 bis 3 Kohlenstoffatomen verstanden, soweit sie mindestens eine Dreifachbindung aufweisen.

Unter dem Begriff "C₃₋₇-Cycloalkyl" (auch soweit sie Bestandteil anderer Reste sind) werden cyclische Alkylgruppen mit 3 bis 7 Kohlenstoffatomen verstanden. Beispielsweise werden hierfür genannt: Cyclopropyl, Cyclopentyl oder Cyclohexyl. Soweit nicht anders beschrieben, können die cyclischen Alkylgruppen substituiert sein mit einem oder mehreren Resten ausgewählt aus der Gruppe bestehend aus Methyl, Ethyl, *iso*-Propyl, *tert*-Butyl, Hydroxy, Fluor, Chlor, Brom und Jod.

Unter dem Begriff "Aryl" (auch soweit sie Bestandteil anderer Reste sind) werden aromatische Ringsysteme mit 6, 10 oder 14 Kohlenstoffatomen verstanden. Beispielsweise werden hierfür genannt: Phenyl oder Naphthyl, bevorzugter Arylrest ist Phenyl. Soweit nicht anders beschreiben, können die Aromaten substituiert sein mit einem oder mehreren Resten ausgewählt aus der Gruppe bestehend aus Methyl, Ethyl, *iso*-Propyl, *tert*-Butyl, Hydroxy, Fluor, Chlor, Brom und Jod.

Die als Ausgangsstoffe verwendeten Verbindungen der allgemeinen Formeln II bis XV sind teilweise literaturbekannt oder können nach an sich literaturbekannten Verfahren (siehe Beispiele I bis II) oder den vorstehend beschriebenen Verfahren, gegebenenfalls unter zusätzlicher Einführung von Schutzresten erhalten werden.

Wie bereits eingangs erwähnt, weisen die erfindungsgemäßen Verbindungen der allgemeinen Formel I und ihre physiologisch verträglichen Salze wertvolle pharmakologische Eigenschaften auf, insbesondere eine Hemmwirkung auf die durch den Epidermal Growth Factor-Rezeptor (EGF-R) vermittelte Signaltransduktion, wobei diese beispielsweise durch eine Inhibition der Ligandenbindung, der Rezeptordimerisierung oder der Tyrosinkinase selbst bewirkt werden kann. Außerdem ist es möglich, daß die Signalübertragung an weiter abwärtsliegenden Komponenten blockiert wird.

Die biologischen Eigenschaften der neuen Verbindungen werden beispielsweise wie folgt geprüft:

Die Hemmung der EGF-R vermittelten Signalübertragung kann z.B. mit Zellen nachgewiesen werden, die humanen EGF-R exprimieren und deren Überleben und Proliferation von Stimulierung durch EGF bzw. TGF-alpha abhängt. Eine murine hämatopoetische Zelllinie wird derart genetisch verändert, dass sie funktionellen humanen EGF-R exprimiert. Die Proliferation dieser Zelllinie kann daher durch EGF stimuliert werden.

Der Test wird wie folgt durchgeführt:

Die Zellen werden in RPMI/1640 Medium kultiviert. Die Proliferation wird mit 20 ng/ml humanem EGF (Promega) stimuliert. Zur Untersuchung der inhibitorischen Aktivität der erfindungsgemäßen Verbindungen werden diese Verbindungen in 100% Dimethylsulfoxid (DMSO) gelöst und in verschiedenen Verdünnungen den Kulturen zugefügt, wobei die maximale DMSO Konzentration 1 % beträgt. Die Kulturen werden für 48 Stunden bei 37°C inkubiert.

Zur Bestimmung der inhibitorischen Aktivität der erfindungsgemäßen Verbindungen wird die relative Zellzahl mit dem Cell Titer 96TM AQueous Non-Radioactive Cell Proliferation Assay (Promega) in O.D. Einheiten gemessen. Die relative Zellzahl wird in Prozent der Kontrolle berechnet und die Wirkstoffkonzentration, die die Proliferation der Zellen zu 50% hemmt (IC50), abgeleitet.
Die erfindungsgemäßen Verbindungen der allgemeinen Formel I zeigen beispielsweise IC50-Werte von < 10 micromolar, vorzugsweise von < 1 micromolar.

Die erfindungsgemäßen Verbindungen der allgemeinen Formel I hemmen somit die Signaltransduktion durch Tyrosinkinasen, wie am Beispiel des humanen EGF-Rezeptors gezeigt wurde, und sind daher nützlich zur Behandlung pathophysiologischer Prozesse, die durch Überfunktion von Tyrosinkinasen hervorgerufen werden. Das sind z.B. benigne oder maligne Tumoren, insbesondere Tumoren epithelialen und neuroepithelialen Ursprungs, Metastasierung sowie die abnorme Proliferation vaskulärer Endothelzellen (Neoangiogenese).

Die erfindungsgemäßen Verbindungen sind auch nützlich zur Vorbeugung und Behandlung von Erkrankungen der Atemwege und der Lunge, die mit einer vermehrten oder veränderten Schleimproduktion einhergehen, die durch Stimulation von Tyrosinkinasen hervorgerufen wird, wie z.B. bei entzündlichen Erkrankungen der Atemwege wie chronische Bronchitis, chronisch obstruktive Bronchitis, Asthma, Bronchiektasien, allergische oder nicht-allergische Rhinitis oder Sinusitis, zystische Fibrose, α1-Antitrypsin-Mangel, oder bei Husten, Lungenemphysem, Lungenfibrose und hyperreaktiven Atemwegen.

Die Verbindungen sind auch geeignet für die Behandlung von Erkrankungen des Magen-Darm-Traktes und der Gallengänge und -blase, die mit einer gestörten Aktivität der Tyrosinkinasen einhergehen, wie sie z.B. bei chronisch entzündlichen Veränderungen zu finden sind, wie Cholezystitis, M. Crohn, Colitis ulcerosa, und Geschwüren im Magen-Darm-Trakt oder wie sie bei Erkrankungen des Magen-Darm-Traktes, die mit einer vermehrten Sekretion einhergehen, vorkommen, wie M. Ménétrier, sezernierende Adenome und Proteinverlustsyndrome.

Außerdem können die Verbindungen der allgemeinen Formel I und deren physiologisch verträglichen Salze zur Behandlung anderer Krankheiten verwendet werden, die durch aberrante Funktion von Tyrosinkinasen verursacht werden, wie z.B. epidermaler Hyperproliferation (Psoriasis), benigner Prostatahyperplasie (BPH), inflammatorischer Prozesse, Erkrankungen des Immunsystems, Hyperproliferation hämatopoetischer Zellen, der Behandlung von Nasenpolypen, etc..

Auf Grund ihrer biologischen Eigenschaften können die erfindungsgemäßen Verbindungen allein oder in Kombination mit anderen pharmakologisch wirksamen Verbindungen angewendet werden, beispielsweise in der Tumortherapie in Monotherapie oder in Kombination mit anderen Anti-Tumor Therapeutika, beispielsweise in Kombination mit Topoisomerase-Inhibitoren (z.B. Etoposide), Mitoseinhibitoren (z.B. Vinblastin), mit Nukleinsäuren interagierenden Verbindungen (z.B. cis-Platin, Cyclophosphamid, Adriamycin), Hormon-Antagonisten (z.B. Tamoxifen), Inhibitoren metabolischer Prozesse (z.B. 5-FU etc.), Zytokinen (z.B. Interferonen), Antikörpern etc. Für die Behandlung von Atemwegserkrankungen können diese Verbindungen allein oder in Kombination mit anderen Atemwegstherapeutika, wie z.B. sekretolytisch (z.B. Ambroxol, N-acetylcystein), broncholytisch (z.B. Tiotropium oder lpratropium oder Fenoterol, Salmeterol, Salbutamol) und/oder entzündungshemmend (z.B. Theophylline oder Glucocorticoide) wirksamen Substanzen angewendet werden. Für die Behandlung von Erkrankungen im Bereich des Magen-Darm-Traktes können diese Verbindungen ebenfalls alleine oder in Kombination mit Motilitäts- oder Sekretions-beeinflussenden Substanzen gegeben werden. Diese Kombinationen können entweder simultan oder sequentiell verabreicht werden.

Die Anwendung dieser Verbindungen entweder alleine oder in Kombination mit anderen Wirkstoffen kann intravenös, subkutan, intramuskulär, intraperitoneal, intranasal, durch Inhalation oder transdermal oder oral erfolgen, wobei zur Inhalation insbesondere Aerosolformulierungen geeignet sind.

Bei der pharmazeutischen Anwendung werden die erfindungsgemäßen Verbindungen in der Regel bei warmblütigen Wirbeltieren, insbesondere beim Menschen, in Dosierungen von 0.01-100 mg/kg Körpergewicht, vorzugsweise bei 0.1-15 mg/kg verwendet. Zur Verabreichung werden diese mit einem oder mehreren üblichen inerten Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Stearylalkohol, Carboxymethylcellulose oder fetthaltigen Substanzen wie Hartfett oder deren geeigneten Gemischen in übliche galenische Zubereitungen wie Tabletten, Dragees, Kapseln, Pulver, Suspensionen, Lösungen, Sprays oder Zäpfchen eingearbeitet.

Die nachfolgenden Beispiele sollen die vorliegende Erfindung näher erläutern ohne diese zu beschränken:
Herstellung der Ausgangsverbindungen:

### Beispiel I

### 4-[(3-Chlor-2-fluor-phenyl)amino]-6-(4-oxo-cyclohexyloxy)-7-methoxy-chinazolin

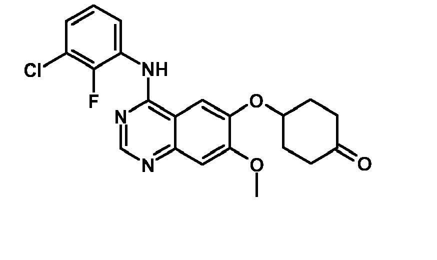

Zu 9.0 g 4-[(3-Chlor-2-fluor-phenyl)amino]-6-(1,4-dioxa-spiro[4.5]decan-8-yl-oxy)-7-methoxy-chinazolin in 110 ml Tetrahydrofuran werden 25 ml 4M Schwefelsäure gegeben und das Gemisch 18 Stunden bei Raumtemperatur gerührt. Das Gemisch wird mit 4M Natronlauge alkalisch gestellt und mehrmals mit Essigester extrahiert. Die vereinigten organischen Phasen werden getrocknet, eingeengt und mit Diethylether verrührt. Der Feststoff wird abgesaugt und getrocknet.
Ausbeute: 7.4 g (90 % der Theorie)
Massenspektrum (ESI⁺): m/z = 416, 418 [M+H]⁺

### Beispiel II

### 4-[(3-Chlor-2-fluor-phenyl)amino]-6-(1,4-dioxa-spiro[4.5]decan-8-yl-oxy)-7-methoxy-chinazolin

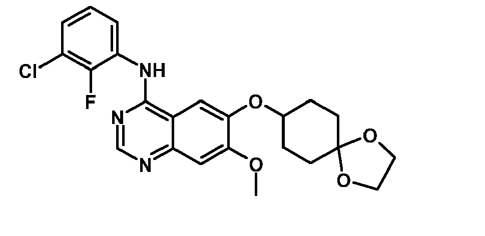

Zu 18.1 g 4-[(3-Chlor-2-fluor-phenyl)amino]-6-hydroxy-7-methoxy-chinazolin (siehe beispielsweise Bioorganic & Medicinal Chemistry Letters (2006), 16(18), 4908-4912)
in 125 ml Dimethylformamid werden bei 50°C 12.5 g Kaliumcarbonat und 16 g 8-Methansulfonyloxy-1,4-dioxa-spiro[4.5]decan (siehe beispielsweise Journal of Medicinal Chemistry (1992), 35(12), 2243-7) gegeben und das Gemisch 18 Stunden bei 80°C gerührt. Es werden nochmals 4.7 g Kaliumcarbonat und 4.0 g 8-Methansulfonyloxy-1,4-dioxa-spiro[4.5]decan zugegeben und weitere 7 Stunden bei 80°C gerührt. Das Reaktionsgemisch wird abgekühlt, mit Wasser und Essigester verdünnt und der gebildete Niederschlag wird abgesaugt und getrocknet.
Ausbeute: 12.2 g (47 % der Theorie)
Massenspektrum (ESI⁺): m/z = 460, 462 [M+H]⁺

### Herstellung der Endverbindungen:

### Beispiel 1

### 4-[(3-Chlor-2-fluor-phenyl)amino]-6-[cis-4-(morpholin-4-yl)-cyclohexyloxy]-7-methoxy-chinazolin und 4-[(3-Chlor-2-fluor-phenyl)amino]-6-[trans-4-(morpholin-4-yl)-cyclohexyloxy]-7-methoxy-chinazolin

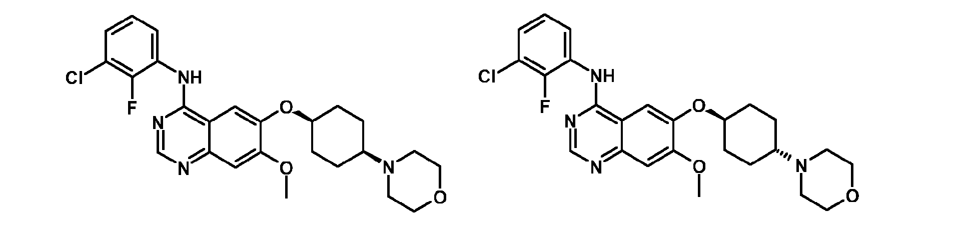

Zu 800 mg 4-[(3-Chlor-2-fluor-phenyl)amino]-6-(4-oxo-cyclohexyloxy)-7-methoxy-chinazolin in 25 ml 1,2-Dichlorethan werden mit 175 µl Morpholin, 600 mg Natrium-triacetoxyborhydrid und 115 µl Eisessig zugesetzt und das Gemisch 18 Stunden bei Raumtemperatur unter einer Argon-Atmosphäre gerührt. Es wird noch etwas Natrium-triacetoxyborhydrid nachgesetzt und weitere 3 Stunden gerührt. Das Reaktionsgemisch wird mit 1M Natronlauge versetzt und kurz gerührt, anschließend wird mit Dichlormethan mehrfach extrahiert. Die vereinigten organischen Phasen werden über Magnesiumsulfat getrocknet und eingeengt. Durch Reinigung über eine Kieselgelsäule mit Dichlormethan/Methanol (99:1 bis 80:20) werden die beiden Titelverbindungen als Gemisch erhalten. Die Auftrennung des cis/trans-Gemisches erfolgt durch präparative HPLC (xBridge™ C18 der Fa. Waters; Acetonitril, Wasser, wässriges Ammoniak). Die Zuordnung der Isomeren erfolgt mittels 1H-NMR-Spektroskopie.

4-[(3-Chlor-2-fluor-phenyl)amino]-6-[cis-4-(morpholin-4-yl)-cyclohexyloxy]-7-methoxy-chinazolin:
Ausbeute: 250 mg (25 % der Theorie)
Massenspektrum (ESI⁺): m/z = 487, 489 [M+H]⁺

4-[(3-Chlor-2-fluor-phenyl)amino]-6-[trans-4-(morpholin-4-yl)-cyclohexyloxy]-7-methoxy-chinazolin:
Ausbeute: 320 mg (33 % der Theorie)
Massenspektrum (ESI⁺): m/z = 487, 489 [M+H]⁺

Analog Beispiel 1 werden folgende Verbindungen erhalten:

### (1) 4-[(3-Chlor-2-fluor-phenyl)amino]-6-[(R)-cis-4-(3-hydroxy-pyrrolidin-1-yl)-cyclohexyloxy]-7-methoxy-chinazolin und 4-[(3-Chlor-2-fluor-phenyl)amino]-6-[(R)-trans-4-(3-hydroxy-pyrrolidin-1-yl)-cyclohexyloxy]-7-methoxy-chinazolin

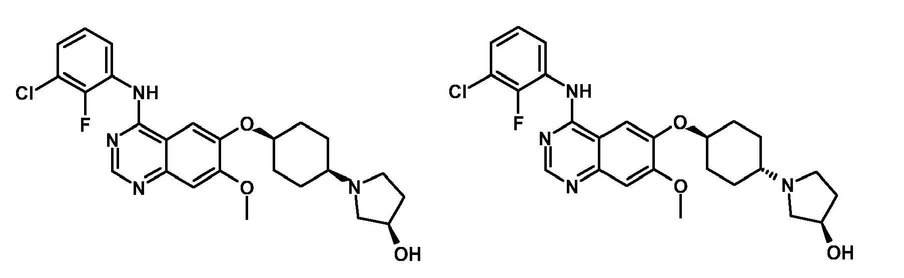

Die Reaktion wird in Tetrahydrofuran durchgeführt.

4-[(3-Chlor-2-fluor-phenyl)amino]-6-[(R)-cis-4-(3-hydroxy-pyrrolidin-1-yl)-cyclohexyloxy]-7-methoxy-chinazolin
Massenspektrum (ESI⁺): m/z = 487, 489 [M+H]⁺

4-[(3-Chlor-2-fluor-phenyl)amino]-6-[(R)-trans-4-(3-hydroxy-pyrrolidin-1-yl)-cyclohexyloxy]-7-methoxy-chinazolin
Massenspektrum (ESI⁺): m/z = 487, 489 [M+H]⁺

### (2) 4-[(3-Chlor-2-fluor-phenyl)amino]-6-[(S)-cis-4-(3-hydroxy-pyrrolidin-1-yl)-cyclohexyloxy]-7-methoxy-chinazolin und 4-[(3-Chlor-2-fluor-phenyl)amino]-6-[(S)-trans-4-(3-hydroxy-pyrrolidin-1-yl)-cyclohexyloxy]-7-methoxy-chinazolin

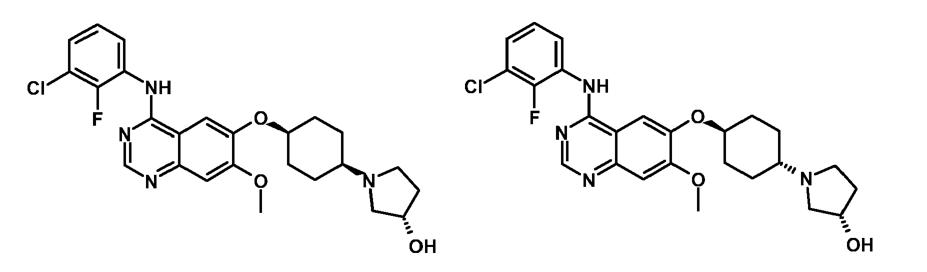

Die Reaktion wird in Tetrahydrofuran durchgeführt.

4-[(3-Chlor-2-fluor-phenyl)amino]-6-[(S)-cis-4-(3-hydroxy-pyrrolidin-1-yl)-cyclohexyloxy]-7-methoxy-chinazolin
Massenspektrum (ESI⁺): m/z = 487, 489 [M+H]⁺

4-[(3-Chlor-2-fluor-phenyl)amino]-6-[(S)-trans-4-(3-hydroxy-pyrrolidin-1-yl)-cyclohexyloxy]-7-methoxy-chinazolin
Massenspektrum (ESI⁺): m/z = 487, 489 [M+H]⁺

### (3) 4-[(3-Chlor-2-fluor-phenyl)amino]-6-[cis-4-(3-oxo-piperazin-1-yl)-cyclohexyloxy]-7-methoxy-chinazolin und 4-[(3-Chlor-2-fluor-phenyl)amino]-6-[trans-4-(3-oxo-piperazin-1-yl)-cyclohexyloxy]-7-methoxy-chinazolin

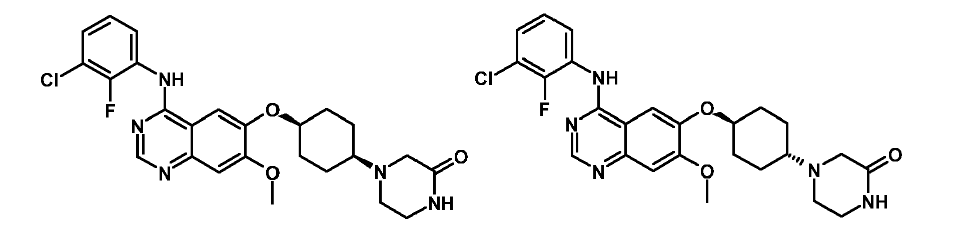

Die Reaktion wird in 1,2-Dichlorethan durchgeführt.

4-[(3-Chlor-2-fluor-phenyl)amino]-6-[cis-4-(3-oxo-piperazin-1-yl)-cyclohexyloxy]-7-methoxy-chinazolin
Massenspektrum (ESI⁺): m/z = 500, 502 [M+H]⁺

4-[(3-Chlor-2-fluor-phenyl)amino]-6-[trans-4-(3-oxo-piperazin-1-yl)-cyclohexyloxy]-7-methoxy-chinazolin
Massenspektrum (ESI⁺): m/z = 500, 502 [M+H]⁺

### (4) 4-[(3-Chlor-2-fluor-phenyl)amino]-6-{(S)-cis-4-[2-(N,N-dimethylaminocarbonyl)-pyrrolidin-1-yl]-cyclohexyloxy]-7-methoxy-chinazolin und 4-[(3-Chlor-2-fluor-phenyl)amino]-6-{(S)-trans-4-[2-(N, N-dimethylaminocarbonyl)-pyrrolidin-1-yl]-cyclohexyloxy]-7-methoxy-chinazolin

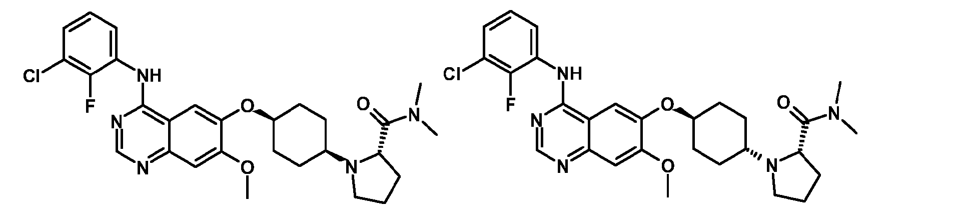

Die Reaktion wird in Tetrahydrofuran durchgeführt.

4-[(3-Chlor-2-fluor-phenyl)amino]-6-{(S)-cis-4-[2-(N, N-dimethylaminocarbonyl)-pyrrolidin-1-yl]-cyclohexyloxy]-7-methoxy-chinazolin
Massenspektrum (ESI⁺): m/z = 542, 544 [M+H]⁺

4-[(3-Chlor-2-fluor-phenyl)amino]-6-{(S)-trans-4-[2-(N,N-dimethylaminocarbonyl)-pyrrolidin-1-yl]-cyclohexyloxy]-7-methoxy-chinazolin
Massenspektrum (ESI⁺): m/z = 542, 544 [M+H]⁺

### (5) 4-[(3-Chlor-2-fluor-phenyl)amino]-6-{(S)-cis-4-[2-(aminocarbonyl)-pyrrolidin-1-yl]-cyclohexyloxy]-7-methoxy-chinazolin und 4-[(3-Chlor-2-fluor-phenyl)amino]-6-{(S)-trans-4-[2-(aminocarbonyl)-pyrrolidin-1-yl]-cyclohexyloxy]-7-methoxy-chinazolin

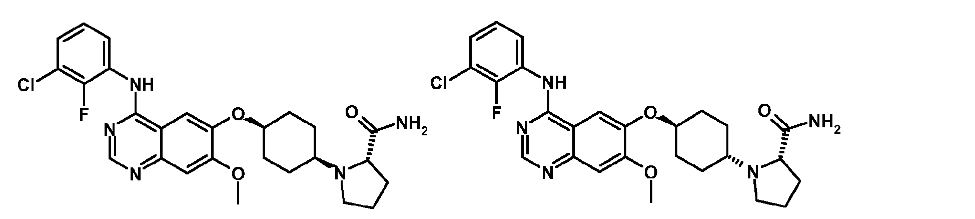

Die Reaktion wird in Tetrahydrofuran durchgeführt.

4-[(3-Chlor-2-fluor-phenyl)amino]-6-{(S)-cis-4-[2-(aminocarbonyl)-pyrrolidin-1-yl]-cyclohexyloxy]-7-methoxy-chinazolin
Massenspektrum (ESI⁺): m/z = 514, 516 [M+H]⁺

4-[(3-Chlor-2-fluor-phenyl)amino]-6-{(S)-trans-4-[2-(aminocarbonyl)-pyrrolidin-1-yl]-cyclohexyloxy]-7-methoxy-chinazolin
Massenspektrum (ESI⁺): m/z = 514, 516 [M+H]⁺

Analog den vorstehend genannten Beispielen und anderen literaturbekannten Verfahren können auch folgende Verbindungen hergestellt werden:

| **Beispiel-Nr.** | **Struktur** |
|---|---|
| (1) | 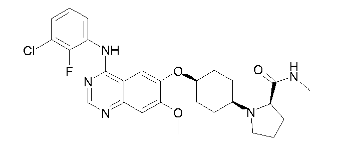 |
| (2) | 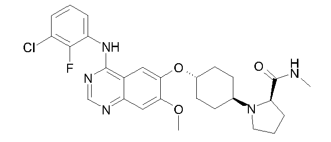 |
| (3) | 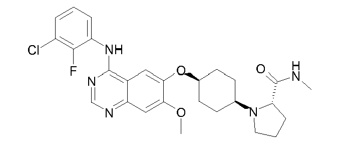 |
| (4) | 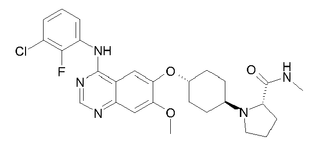 |
| (5) | 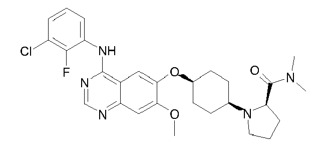 |
| (6) | 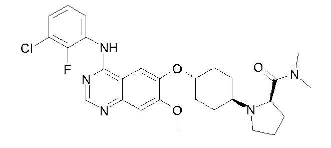 |
| (7) | 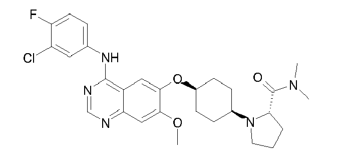 |
| (8) | 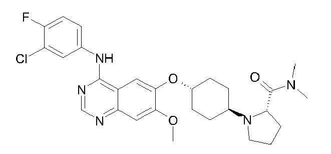 |
| (9) | 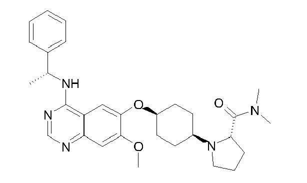 |
| (10) | 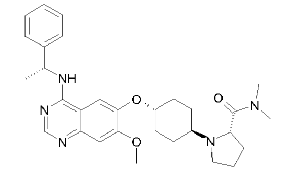 |
| (11) | 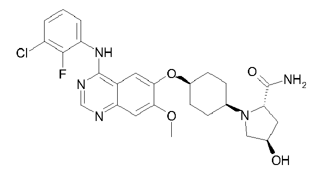 |
| (12) | 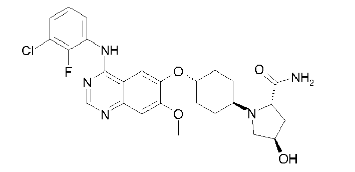 |
| (13) | 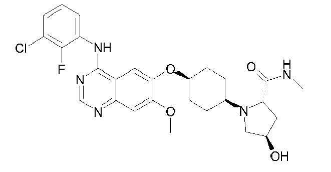 |
| (14) | 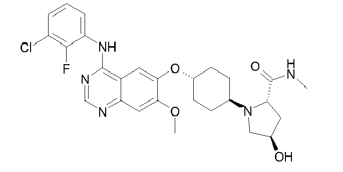 |
| (15) | 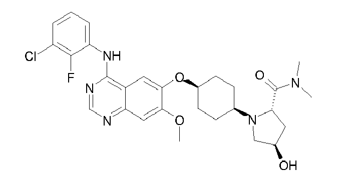 |
| (16) | 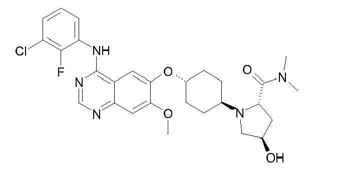 |
| (17) | 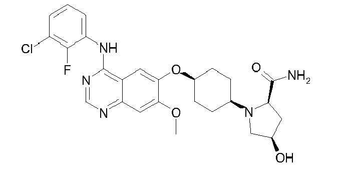 |
| (18) | 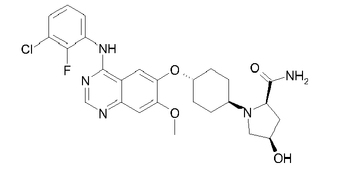 |
| (19) | 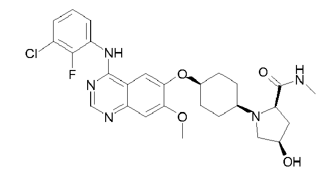 |
| (20) | 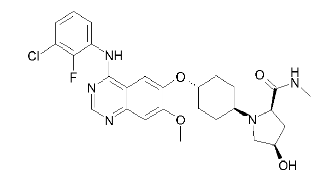 |
| (21) | 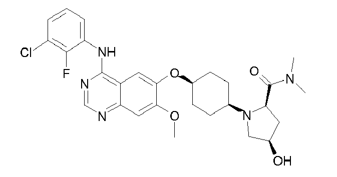 |
| (22) | 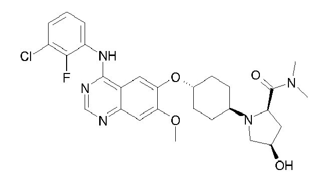 |
| (23) | 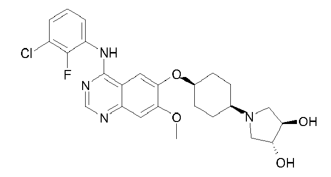 |
| (24) | 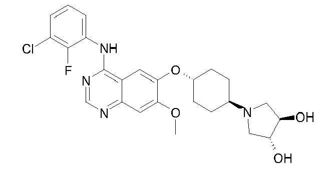 |
| (25) | 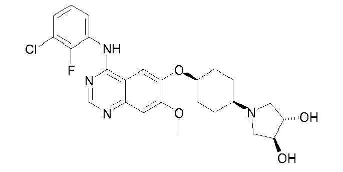 |
| (26) | 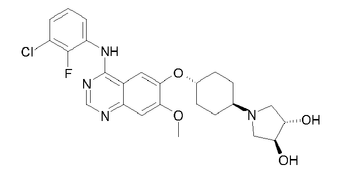 |
| (27) | 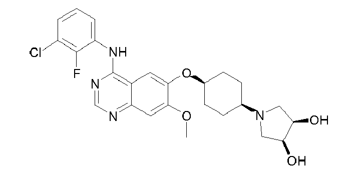 |
| (28) | 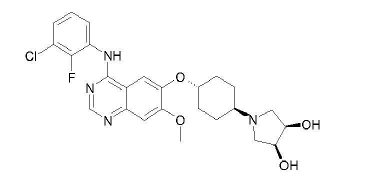 |
| (29) | 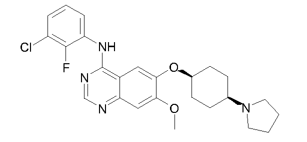 |
| (30) | 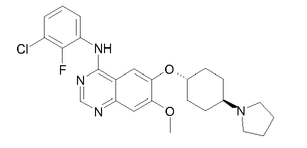 |
| (31) | 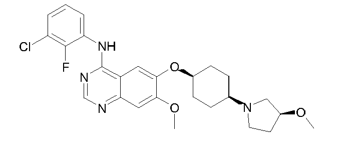 |
| (32) | 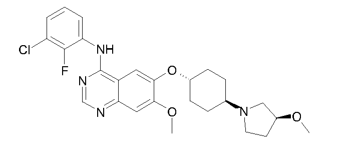 |
| (33) | 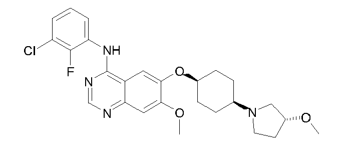 |
| (34) | 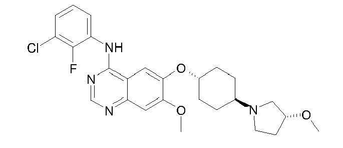 |
| (35) | 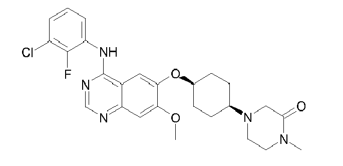 |
| (36) | 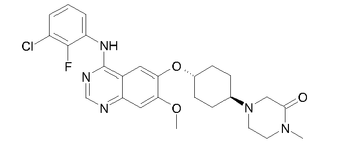 |
| (37) | 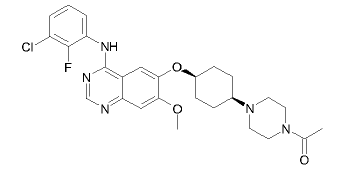 |
| (38) | 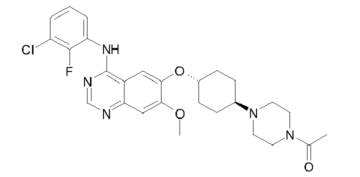 |
| (39) | 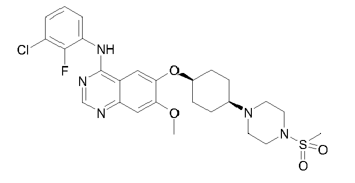 |
| (40) | 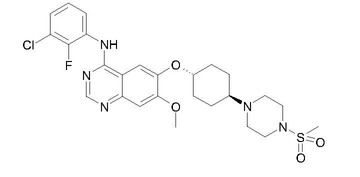 |
| (41) | 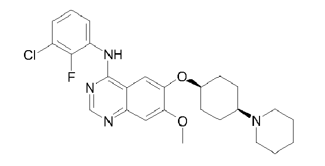 |
| (42) | 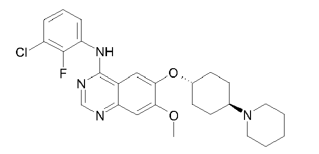 |
| (43) | 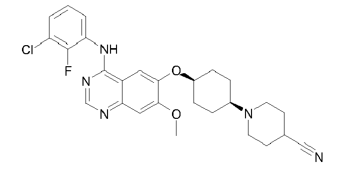 |
| (44) | 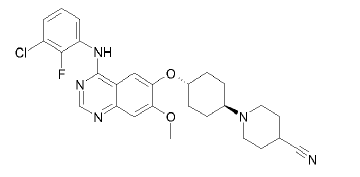 |
| (45) | 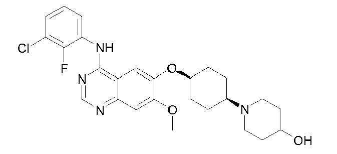 |
| (46) | 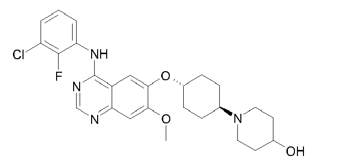 |
| (47) | 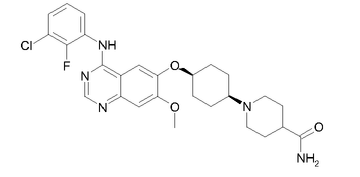 |
| (48) | 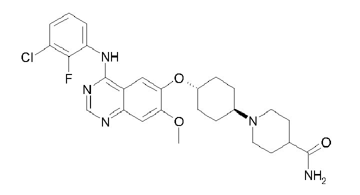 |
| (49) | 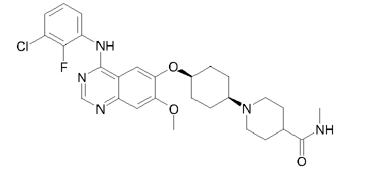 |
| (50) | 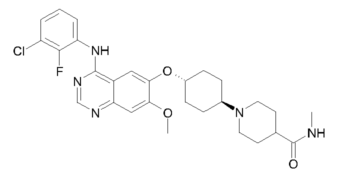 |
| (51) | 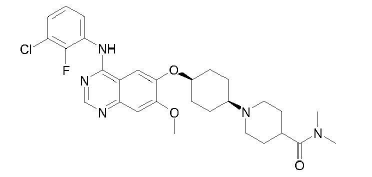 |
| (52) | 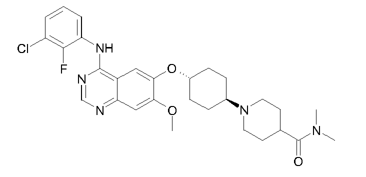 |
| (53) | 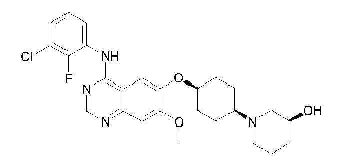 |
| (54) | 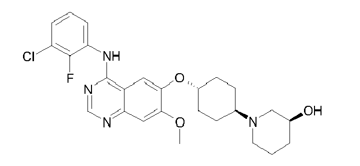 |
| (55) | 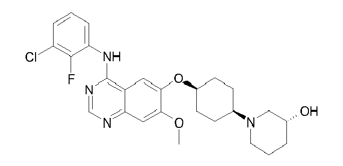 |
| (56) | 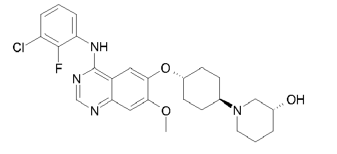 |
| (57) | 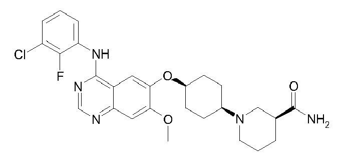 |
| (58) | 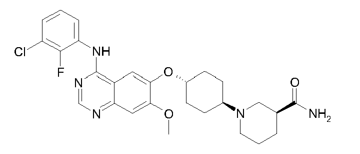 |
| (59) | 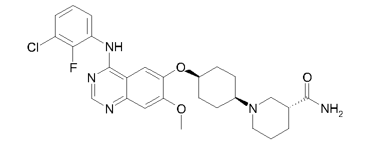 |
| (60) | 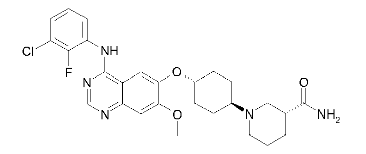 |
| (61) | 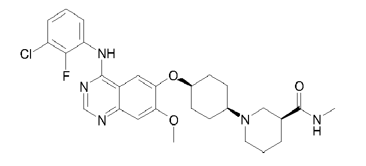 |
| (62) | 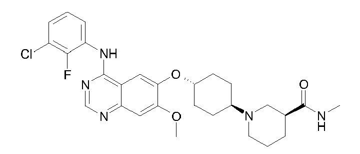 |
| (63) | 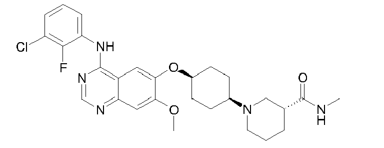 |
| (64) | 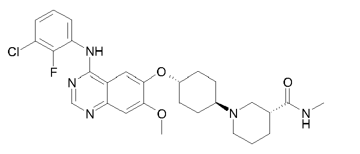 |
| (65) | 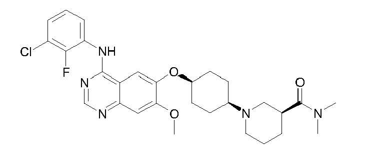 |
| (66) | 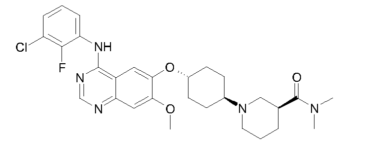 |
| (67) | 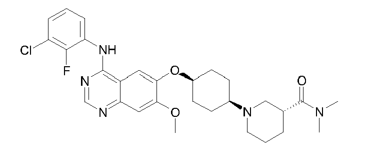 |
| (68) | 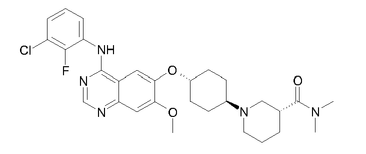 |
| (69) | 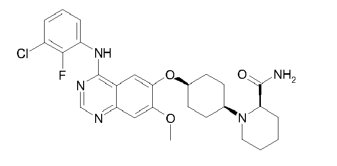 |
| (70) | 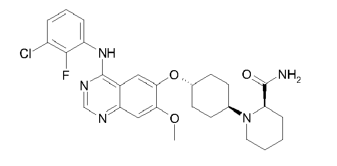 |
| (71) | 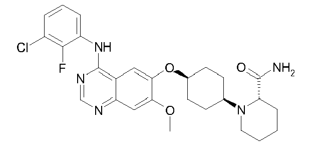 |
| (72) | 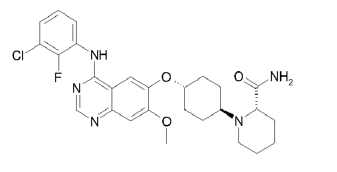 |
| (73) | 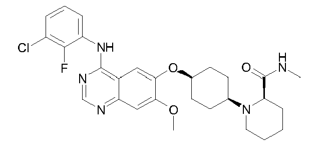 |
| (74) | 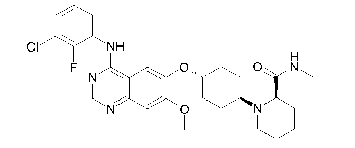 |
| (75) | 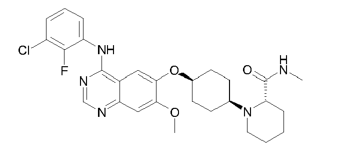 |
| (76) | 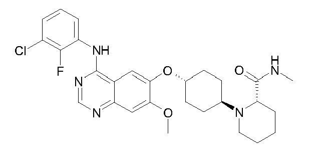 |
| (77) | 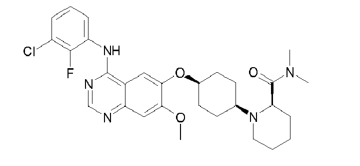 |
| (78) | 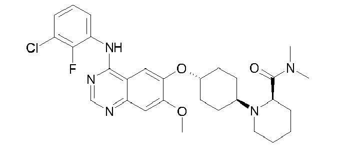 |
| (79) | 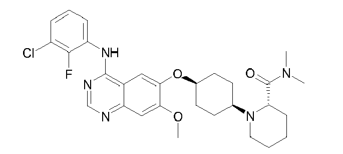 |
| (80) | 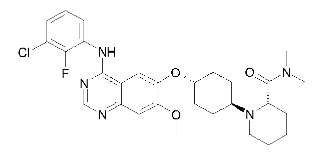 |
| (81) | 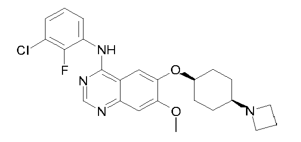 |
| (82) | 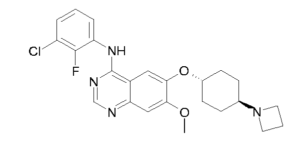 |
| (83) | 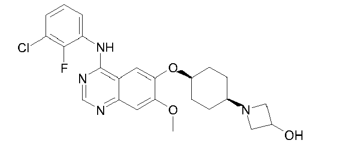 |
| (84) | 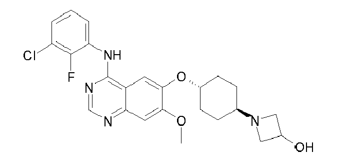 |
| (85) | 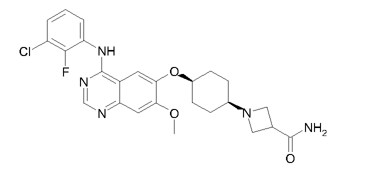 |
| (86) | 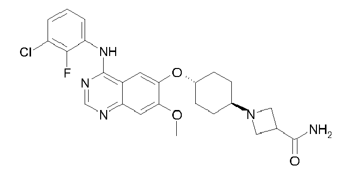 |
| (87) | 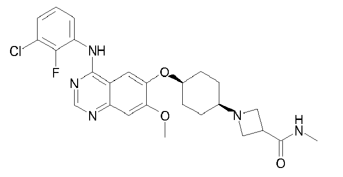 |
| (88) | 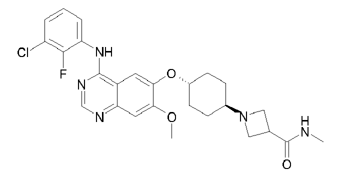 |
| (89) | 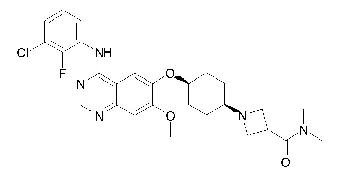 |
| (90) | 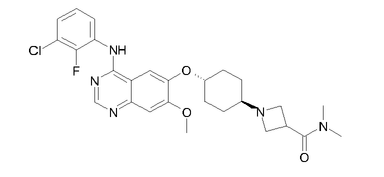 |
| (91) | 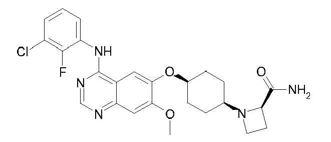 |
| (92) | 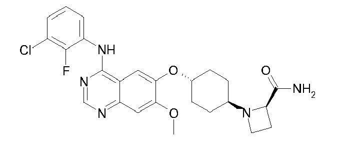 |
| (93) | 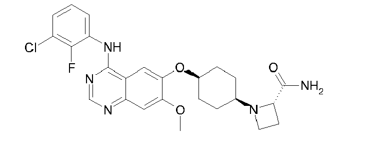 |
| (94) | 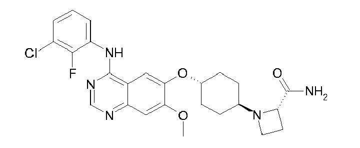 |
| (95) | 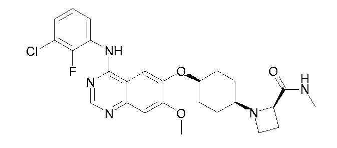 |
| (96) | 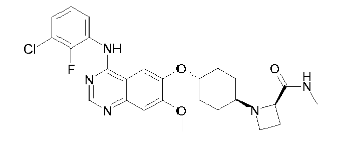 |
| (97) | 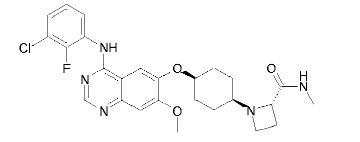 |
| (98) | 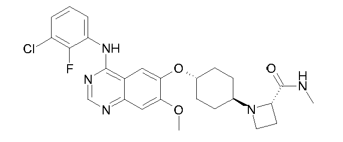 |
| (99) | 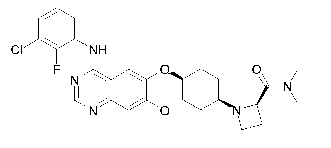 |
| (100) | 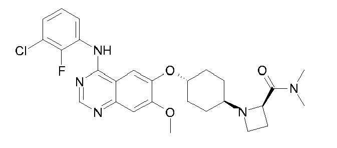 |
| (101) | 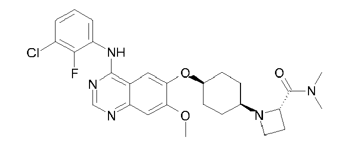 |
| (102) | 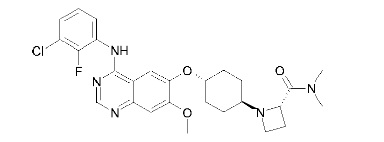 |

### Beispiel 2

### Dragees mit 75 mg Wirksubstanz

### Zusammensetzung:

| | | |
|---|---|---|
| 1 | Dragéekern enthält: | |
| | Wirksubstanz | 75.0 mg |
| | Calciumphosphat | 93.0 mg |
| | Maisstärke | 35.5 mg |
| | Polyvinylpyrrolidon | 10.0 mg |
| | Hydroxypropylmethylcellulose | 15.0 mg |
| | Magnesiumstearat | 1.5 mg |
| | | 230.0 mg |

### Herstellung:

Die Wirksubstanz wird mit Calciumphosphat, Maisstärke, Polyvinylpyrrolidon, Hydroxypropylmethylcellulose und der Hälfte der angegebenen Menge Magnesiumstearat gemischt. Auf einer Tablettiermaschine werden Preßlinge mit einem Durchmesser von ca. 13 mm hergestellt, diese werden auf einer geeigneten Maschine durch ein Sieb mit 1.5 mm-Maschenweite gerieben und mit der restlichen Menge Magnesiumstearat vermischt. Dieses Granulat wird auf einer Tablettiermaschine zu Tabletten mit der gewünschten Form gepreßt.

| | | |
|---|---|---|
| Kerngewicht: | 230 | mg |
| Stempel: | 9 | mm, gewölbt |

Die so hergestellten Dragéekerne werden mit einem Film überzogen, der im wesentlichen aus Hydroxypropylmethylcellulose besteht. Die fertigen Filmdragees werden mit Bienenwachs geglänzt.

| | |
|---|---|
| Dragéegewicht: | 245 mg. |

### Beispiel 3

### Tabletten mit 100 mg Wirksubstanz

### Zusammensetzung:

| | | |
|---|---|---|
| 1 | Tablette enthält: | |
| | Wirksubstanz | 100.0 mg |
| | Milchzucker | 80.0 mg |
| | Maisstärke | 34.0 mg |
| | Polyvinylpyrrolidon | 4.0 mg |
| | Magnesiumstearat | 2.0 mg |
| | | 220.0 mg |

### Herstellungverfahren:

Wirkstoff, Milchzucker und Stärke werden gemischt und mit einer wäßrigen Lösung des Polyvinylpyrrolidons gleichmäßig befeuchtet. Nach Siebung der feuchten Masse (2.0 mm-Maschenweite) und Trocknen im Hordentrockenschrank bei 50°C wird erneut gesiebt (1.5 mm-Maschenweite) und das Schmiermittel zugemischt. Die preßfertige Mischung wird zu Tabletten verarbeitet.

| | | |
|---|---|---|
| Tablettengewicht: | 220 mg | |
| Durchmesser: | 10 mm, | biplan mit beidseitiger Facette und einseitiger Teilkerbe. |

### Beispiel 4

### Tabletten mit 150 mg Wirksubstanz

### Zusammensetzung:

| | |
|---|---|
| 1 | Tablette enthält: |
| Wirksubstanz | 150.0 mg |
| Milchzucker pulv. | 89.0 mg |
| Maisstärke | 40.0 mg |
| Kolloide Kieselgelsäure | 10.0 mg |
| Polyvinylpyrrolidon | 10.0 mg |
| Magnesiumstearat | 1.0 mg |
| | 300.0 mg |

### Herstellung:

Die mit Milchzucker, Maisstärke und Kieselsäure gemischte Wirksubstanz wird mit einer 20%igen wäßrigen Polyvinylpyrrolidonlösung befeuchtet und durch ein Sieb mit 1.5 mm-Maschenweite geschlagen.
Das bei 45°C getrocknete Granulat wird nochmals durch dasselbe Sieb gerieben und mit der angegebenen Menge Magnesiumstearat gemischt. Aus der Mischung werden Tabletten gepreßt.

| | |
|---|---|
| Tablettengewicht: | 300 mg |
| Stempel: | 10 mm, flach |

### Beispiel 5

### Hartgelatine-Kapseln mit 150 mg Wirksubstanz

### Zusammensetzung:

| | | | |
|---|---|---|---|
| 1 | Kapsel enthält: | | |
| | Wirkstoff | | 150.0 mg |
| | Maisstärke getr. | ca. | 180.0 mg |
| | Milchzucker pulv. | ca. | 87.0 mg |
| | Magnesiumstearat | | 3.0 mg |
| | ca. | | 420.0 mg |

### Herstellung:

Der Wirkstoff wird mit den Hilfsstoffen vermengt, durch ein Sieb von 0.75 mm-Maschenweite gegeben und in einem geeigneten Gerät homogen gemischt.
Die Endmischung wird in Hartgelatine-Kapseln der Größe 1 abgefüllt.
Kapselfüllung: ca. 320 mg
Kapselhülle: Hartgelatine-Kapsel Größe 1.

### Beispiel 6

### Suppositorien mit 150 mg Wirksubstanz

### Zusammensetzung:

| | | |
|---|---|---|
| 1 | Zäpfchen enthält: | |
| | Wirkstoff | 150.0 mg |
| | Polyäthylenglykol 1500 | 550.0 mg |
| | Polyäthylenglykol 6000 | 460.0 mg |
| | Polyoxyäthylensorbitanmonostearat | 840.0 mg |
| | | 2000.0 mg |

### Herstellung:

Nach dem Aufschmelzen der Suppositorienmasse wird der Wirkstoff darin homogen verteilt und die Schmelze in vorgekühlte Formen gegossen.

### Beispiel 7

### Suspension mit 50 mg Wirksubstanz

### Zusammensetzung:

| | | |
|---|---|---|
| 100 ml | Suspension enthalten: | |
| | Wirkstoff | 1.00 g |
| | Carboxymethylcellulose-Na-Salz | 0.10 g |
| | p-Hydroxybenzoesäuremethylester | 0.05 g |
| | p-Hydroxybenzoesäurepropylester | 0.01 g |
| | Rohrzucker | 10.00 g |
| | Glycerin | 5.00 g |
| | Sorbitlösung 70%ig | 20.00 g |
| | Aroma | 0.30 g |
| | Wasser dest.ad 100.00 ml | |

### Herstellung:

Destilliertes Wasser wird auf 70°C erhitzt. Hierin wird unter Rühren p-Hydroxybenzoesäuremethylester und -propylester sowie Glycerin und Carboxymethylcellulose-Natriumsalz gelöst. Es wird auf Raumtemperatur abgekühlt und unter Rühren der Wirkstoff zugegeben und homogen dispergiert. Nach Zugabe und Lösen des Zuckers, der Sorbitlösung und des Aromas wird die Suspension zur Entlüftung unter Rühren evakuiert.
5 ml Suspension enthalten 50 mg Wirkstoff.

### Beispiel 8

### Ampullen mit 10 mg Wirksubstanz

### Zusammensetzung:

| | |
|---|---|
| Wirkstoff | 10.0 mg |
| 0.01 n Salzsäure s.q. | |
| Aqua bidest ad | 2.0 ml |

### Herstellung:

Die Wirksubstanz wird in der erforderlichen Menge 0.01 n HCl gelöst, mit Kochsalz isotonisch gestellt, sterilfiltriert und in 2 ml Ampullen abgefüllt.

### Beispiel 9

### Ampullen mit 50 mg Wirksubstanz

### Zusammensetzung :

| | |
|---|---|
| Wirkstoff | 50.0 mg |
| 0.01 n Salzsäure s.q. | |
| Aqua bidest | ad 10.0 ml |

### Herstellung:

Die Wirksubstanz wird in der erforderlichen Menge 0.01 n HCl gelöst, mit Kochsalz isotonisch gestellt, sterilfiltriert und in 10 ml Ampullen abgefüllt.

### Beispiel 10

### Kapseln zur Pulverinhalation mit 5 mg Wirksubstanz

| 1 Kapsel enthält: | |
|---|---|
| Wirksubstanz | 5.0 mg |
| Lactose für Inhalationszwecke | 15.0 mg |
| | 20.0 mg |

### Herstellung:

Die Wirksubstanz wird mit Lactose für Inhalationszwecke gemischt. Die Mischung wird auf einer Kapselmaschine in Kapseln (Gewicht der Leerkapsel ca. 50 mg) abgefüllt.

| | |
|---|---|
| Kapselgewicht: | 70.0 mg |
| Kapselgröße: | 3 |

### Beispiel 11

### Inhalationslösung für Handvernebler mit 2.5 mg Wirksubstanz

### 1 Hub enthält:

| | |
|---|---|
| Wirksubstanz | 2.500 mg |
| Benzalkoniumchlorid | 0.001 mg |
| 1 N-Salzsäure q.s. | |
| Ethanol/Wasser (50/50) ad | 15.000 mg |

### Herstellung:

Die Wirksubstanz und Benzalkoniumchlorid werden in Ethanol/Wasser (50/50) gelöst. Der pH-Wert der Lösung wird mit 1N-Salzsäure eingestellt. Die eingestellte Lösung wird filtriert und in für den Handvernebler geeignete Behälter (Kartuschen) abgefüllt.

Füllmasse des Behälters: 4.5 g

## Patentansprüche

1. Bicyclische Heterocyclen der allgemeinen Formel 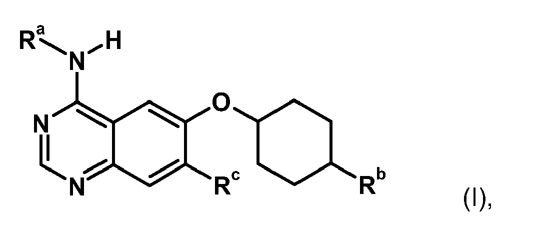 in denen
R^{a} eine Phenyl- oder 1-Phenylethyl-Gruppe, in denen der Phenylkern jeweils durch die Reste R¹ bis R³ substituiert ist, wobei
R¹ und R², die gleich oder verschieden sein können, jeweils ein Wasserstoff-, Fluor-, Chlor-, Brom- oder Jodatom,
eine C₁₋₄-Alkyl-, Hydroxy-, C₁₋₄-Alkoxy-, C₂₋₃-Alkenyl- oder C₂₋₃-Alkinyl-Gruppe,
eine Aryl-, Aryloxy-, Arylmethyl- oder Arylmethoxy-Gruppe,
eine Heteroaryl-, Heteroaryloxy-, Heteroarylmethyl- oder Heteroarylmethoxy-Gruppe,
eine durch 1 bis 3 Fluoratome substituierte Methyl- oder Methoxy-Gruppe oder
eine Cyano-, Nitro- oder Amino-Gruppe, und
R³ ein Wasserstoff-, Fluor-, Chlor- oder Bromatom oder
eine Methyl- oder Trifluormethyl-Gruppe darstellen,
R^{b} eine Azetidin-1-yl-, Pyrrolidin-1-yl-, Piperidin-1-yl-, Homopiperidin-1-yl-, Morpholin-4-yl-, Homomorpholin-4-yl, Piperazin-1-yl-, 4-(C₁₋₄-Alkyl-carbonyl)-piperazin-1-yl-, 4-(C₁₋₄-Alkyl-sulfonyl)-piperazin-1-yl-, Homopiperazin-1-yl-, 4-(C₁₋₄-Alkyl-carbonyl)-homopiperazin-1-yl- oder 4-(C₁₋₄-Alkyl-sulfonyl)-homopiperazin-1-yl-Gruppe, die jeweils durch R⁴ mono-, di- oder trisubstituiert sein kann, wobei die Substituenten gleich oder verschieden sein können und
R⁴ ein Fluor-, Chlor-, Brom- oder lodatom,
eine C₁₋₄-Alkyl-, C₂₋₄-Alkenyl- oder C₂₋₄-Alkinyl-Gruppe,
eine durch 1 bis 3 Fluoratome substituierte Methyl- oder Methoxy-Gruppe,
eine Amino-, C₁₋₄-Alkylamino-, Di-(C₁₋₄-alkyl)amino-, C₁₋₄-Alkyl-carbonylamino-, N-(C₁₋₄-Alkyl)-C₁₋₄-alkyl-carbonylamino-, C₁₋₄-Alkyl-sulfonylamino- oder N-(C₁₋₄-Alkyl)-C₁₋₄-alkyl-sulfonylamino-Gruppe,
eine Amino-C₁₋₄-alkyl-, C₁₋₄-Alkylamino-C₁₋₄-alkyl-, Di-(C₁₋₄-alkyl)amino-C₁₋₄-alkyl-, C₁₋₄-Alkyl-carbonylamino-C₁₋₄-alkyl-, N-(C₁₋₄-Alkyl)-C₁₋₄-alkyl-carbonylamino-C₁₋₄-alkyl-, C₁₋₄-Alkyl-sulfonylamino-C₁₋₄-alkyl- oder N-(C₁₋₄-Alkyl)-C₁₋₄-alkyl-sulfonylamino-C₁₋₄-alkyl-Gruppe,
eine Hydroxy-, C₁₋₄-Alkyloxy- oder C₁₋₄-Alkyl-carbonyloxy-Gruppe,
eine Hydroxy-C₁₋₄-alkyl-, C₁₋₄-Alkyloxy-C₁₋₄-alkyl- oder C₁₋₄-Alkyl-carbonyloxy-C₁₋₄-alkyl-Gruppe,
eine C₁₋₄-Alkyl-carbonyl-, Cyano-, C₁₋₄-Alkyl-oxycarbonyl-, Carboxy-, Aminocarbonyl-, C₁₋₄-Alkyl-aminocarbonyl-, Di-(C₁₋₄-alkyl)amino-carbonyl-, Pyrrolidin-1-yl-carbonyl-, Piperidin-1-yl-carbonyl-, Piperazin-1-yl-carbonyl-, 4-C₁₋₄-Alkyl-piperazin-1-yl-carbonyl- oder Morpholin-4-yl-carbonyl-Gruppe,
eine C₁₋₄-Alkylcarbonyl-C₁₋₄-alkyl-, Cyano-C₁₋₄-alkyl-, C₁₋₄-Alkyloxycarbonyl-C₁₋₄-alkyl-, Aminocarbonyl-C₁₋₄-alkyl-, C₁₋₄-Alkylaminocarbonyl-C₁₋₄-alkyl-, Di-(C₁₋₄-alkyl)aminocarbonyl-C₁₋₄-alkyl-, Pyrrolidin-1-yl-carbonyl-C₁₋₄-alkyl-, Piperidin-1-yl-carbonyl-C₁₋₄-alkyl-, Piperazin-1-yl-carbonyl-C₁₋₄-alkyl-, 4-C₁₋₄-Alkyl-piperazin-1-yl-carbonyl-C₁₋₄-alkyl- oder Morpholin-4-yl-carbonyl-C₁₋₄-alkyl-Gruppe,
eine C₁₋₄-Alkylsulfanyl-, C₁₋₄-Alkylsulfinyl-, C₁₋₄-Alkylsulfonyl-, Aminosulfonyl-, C₁₋₄-Alkyl-aminosulfonyl- oder Di-(C₁₋₄-alkyl)amino-sulfonyl-Gruppe,
eine C₁₋₄-Alkylsulfanyl-C₁₋₄-alkyl-, C₁₋₄-Alkylsulfinyl-C₁₋₄-alkyl-, C₁₋₄-Alkylsulfonyl-C₁₋₄-alkyl-, Aminosulfonyl-C₁₋₄-alkyl-, C₁₋₄-Alkylaminosulfonyl-C₁₋₄-alkyl- oder Di-(C₁₋₄-alkyl)amino-sulfonyl-C₁₋₄-alkyl-Gruppe
darstellt, und wobei die vorstehend erwähnten Heterocylen zusätzlich durch eine Oxo-Gruppe substituiert sein können,
R^{c} ein Wasserstoffatom,
ein Fluor-, Chlor-, Brom- oder lodatom,
eine C₁₋₄-Alkylgruppe,
eine C₁₋₄-Alkylgruppe, die durch einen Rest R⁵ substituiert ist, wobei
R⁵ eine Hydroxy-, C₁₋₃-Alkyloxy-, C₃₋₆-Cycloalkyloxy-, Amino-, C₁₋₃-Alkylamino-, Di-(C₁₋₃-alkyl)amino-, Bis-(2-methoxyethyl)-amino-, Pyrrolidin-1-yl-, Piperidin-1-yl-, Homopiperidin-1-yl-, Morpholin-4-yl-, Homomorpholin-4-yl-, 2-Oxa-5-aza-bicyclo[2.2.1]hept-5-yl-, 3-Oxa-8-aza-bicyclo[3.2.1]oct-8-yl-, 8-Oxa-3-aza-bicyclo[3.2.1]oct-3-yl-, Piperazin-1-yl-, 4-C₁₋₃-Alkyl-piperazin-1-yl-, Homopiperazin-1-yl- oder C₁₋₃-Alkyl-homopiperazin-1-yl-Gruppe oder
eine Formylamino-, C₁₋₄-Alkylcarbonylamino-, C₁₋₃-Alkyloxy-C₁₋₃-alkyl-carbonylamino-, C₁₋₄-Alkyloxycarbonylamino-, Aminocarbonylamino-, C₁₋₃-Alkylaminocarbonylamino-, Di-(C₁₋₃-alkyl)aminocarbonylamino-, Pyrrolidin-1-ylcarbonylamino-, Piperidin-1-ylcarbonylamino-, Piperazin-1-ylcarbonylamino-, 4-C₁₋₃-Alkyl-piperazin-1-ylcarbonylamino-, Morpholin-4-ylcarbonylamino- oder eine C₁₋₄-Alkylsulfonylamino-Gruppe darstellt,
eine Hydroxygruppe,
eine C₁₋₄-Alkyloxygruppe,
eine durch 1 bis 3 Fluoratome substituierte Methoxy- oder Ethyloxy-Gruppe,
eine C₂₋₄-Alkyloxygruppe, die durch den Rest R⁵ substituiert ist, wobei R⁵ wie vorstehend erwähnt definiert ist,
eine C₃₋₇-Cycloalkyloxy- oder C₃₋₇-Cycloalkyl-C₁₋₄-alkyloxy-Gruppe,
eine Tetrahydrofuran-3-yloxy-, Tetrahydropyran-3-yloxy- oder Tetrahydropyran-4-yloxyGruppe,
eine Tetrahydrofuranyl-C₁₋₄-alkyloxy- oder Tetrahydropyranyl-C₁₋₄-alkyloxy-Gruppe,
eine C₁₋₄-Alkoxygruppe, die durch eine in 1-Stellung durch den Rest R⁶ substituierte Pyrrolidinyl-, Piperidinyl- oder Homopiperidinyl-Gruppe substituiert ist, wobei
R⁶ ein Wasserstoffatom oder eine C₁₋₃-Alkylgruppe darstellt,
oder eine C₁₋₄-Alkoxygruppe, die durch eine in 4-Stellung durch den Rest R⁶ substituierte Morpholinylgruppe substituiert ist, wobei R⁶ wie vorstehend erwähnt definiert ist, und wobei die bei der Definition des Restes R^{c} vorstehend erwähnten Pyrrolidinyl-, Piperidinyl-, Piperazinyl- und Morpholinyl-Gruppen jeweils durch eine oder zwei C₁₋₃-Alkylgruppen substituiert sein können, und
wobei unter den bei der Definition der vorstehend genannten Reste erwähnten Arylgruppen jeweils eine Phenylgruppe zu verstehen ist, die durch R⁷ mono- oder disubstituiert ist, wobei die Substituenten gleich oder verschieden sein können und
R⁷ ein Wasserstoffatom, ein Fluor-, Chlor-, Brom- oder lodatom oder eine C₁₋₃-Alkyl-, Hydroxy-, C₁₋₃-Alkyloxy-, Difluormethyl- Trifluormethyl-, Difluormethoxy-, Trifluormethoxy- oder Cyano-Gruppe darstellt, und
unter den bei der Definition der vorstehend genannten Reste erwähnten Heteroarylgruppen eine Pyridyl-, Pyridazinyl-, Pyrimidinyl- oder Pyrazinyl-Gruppe zu verstehen ist, wobei die vorstehend erwähnten Heteroarylgruppen jeweils durch den Rest R⁷ mono- oder disubstituiert sind, wobei die Substituenten gleich oder verschieden sein können und R⁷ wie vorstehend erwähnt definiert ist, und
soweit nichts anderes erwähnt wurde, die vorstehend erwähnten Alkylgruppen geradkettig oder verzweigt sein können,
deren Tautomere, deren Stereoisomere, deren Gemische und deren Salze.

2. Bicyclische Heterocyclen der allgemeinen Formel I gemäß Anspruch 1, in denen
R^{a} eine Phenyl- oder 1-Phenylethyl-Gruppe, in denen der Phenylkern jeweils durch die Reste R¹ bis R³ substituiert ist, wobei
R¹ ein Wasserstoff-, Fluor-, Chlor- oder Bromatom,
eine Methyl-, Trifluormethyl- oder Ethinyl-Gruppe,
eine Phenyloxy- oder Phenylmethoxy-Gruppe, wobei der Phenylteil der vorstehend erwähnten Gruppen gegebenenfalls durch ein Fluor- oder Chloratom substituiert ist, oder
eine Pyridyloxy- oder Pyridinylmethoxy-Gruppe, wobei der Pyridinylteil der vorstehend erwähnten Gruppen gegebenenfalls durch eine Methyl- oder Trifluormethyl-Gruppe substituiert ist,
R² ein Wasserstoff-, Fluor- oder Chloratom oder eine Methylgruppe und
R³ ein Wasserstoffatom darstellen,
R^{b} eine Azetidin-1-yl-, Pyrrolidin-1-yl-, Piperidin-1-yl-, Homopiperidin-1-yl-, Morpholin-4-yl-, Homomorpholin-4-yl, Piperazin-1-yl-, 4-(C₁₋₃-Alkyl-carbonyl)-piperazin-1-yl-, 4-(C₁₋₃-Alkyl-sulfonyl)-piperazin-1-yl-, Homopiperazin-1-yl-, 4-(C₁₋₃-Alkyl-carbonyl)-homopiperazin-1-yl- oder 4-(C₁₋₃-Alkyl-sulfonyl)-homopiperazin-1-yl-Gruppe, die jeweils durch R⁴ mono- oder disubstituiert sein kann, wobei die Substituenten gleich oder verschieden sein können und
R⁴ ein Fluoratom,
eine C₁₋₃-Alkylgruppe,
eine Amino-, C₁₋₃-Alkylamino-, Di-(C₁₋₃-alkyl)amino-, C₁₋₃-Alkyl-carbonylamino-, N-(C₁₋₃-Alkyl)-C₁₋₃-alkyl-carbonylamino-, C₁₋₃-Alkyl-sulfonylamino- oder N-(C₁₋₃-Alkyl)-C₁₋₃-alkyl-sulfonylamino-Gruppe,
eine Amino-C₁₋₃-alkyl-, C₁₋₃-Alkylamino-C₁₋₃-alkyl-, Di-(C₁₋₃-alkyl)amino-C₁₋₃-alkyl-, C₁₋₃-Alkyl-carbonylamino-C₁₋₃-alkyl-, N-(C₁₋₃-Alkyl)-C₁₋₃-alkyl-carbonylamino-C₁₋₃-alkyl-, C₁₋₃-Alkyl-sulfonylamino-C₁₋₃-alkyl- oder N-(C₁₋₃-Alkyl)-C₁₋₃-alkyl-sulfonylamino-C₁₋₃-alkyl-Gruppe,
eine Hydroxy-, C₁₋₃-Alkyloxy- oder C₁₋₃-Alkyl-carbonyloxy-Gruppe,
eine Hydroxy-C₁₋₃-alkyl-, C₁₋₃-Alkyloxy-C₁₋₃-alkyl- oder C₁₋₃-Alkyl-carbonyloxy-C₁₋₃-alkyl-Gruppe,
eine C₁₋₃-Alkyl-carbonyl-, Cyano-, C₁₋₄-Alkyl-oxycarbonyl-, Carboxy-, Aminocarbonyl-, C₁₋₃-Alkyl-aminocarbonyl- oder Di-(C₁₋₃-alkyl)amino-carbonyl-Gruppe,
eine C₁₋₃-Alkylcarbonyl-C₁₋₃-alkyl-, Cyano-C₁₋₃-alkyl-, C₁₋₄-Alkyloxycarbonyl-C₁₋₃-alkyl-gruppe, Aminocarbonyl-C₁₋₃-alkyl-, C₁₋₃-Alkylaminocarbonyl-C₁₋₃-alkyl- oder Di-(C₁₋₃-alkyl)aminocarbonyl-C₁₋₃-alkyl-Gruppe,
eine C₁₋₄-Alkylsulfanyl-, C₁₋₄-Alkylsulfinyl-, C₁₋₄-Alkylsulfonyl-, Aminosulfonyl-, C₁₋₃-Alkyl-aminosulfonyl- oder Di-(C₁₋₃-alkyl)amino-sulfonyl-Gruppe,
eine C₁₋₄-Alkylsulfanyl-C₁₋₃-alkyl-, C₁₋₄-Alkylsulfinyl-C₁₋₃-alkyl-, C₁₋₄-Alkylsulfonyl-C₁₋₃-alkyl-, Aminosulfonyl-C₁₋₃-alkyl-, C₁₋₃-Alkylaminosulfonyl-C₁₋₃-alkyl- oder Di-(C₁₋₃-alkyl)amino-sulfonyl-C₁₋₃-alkyl-Gruppe
darstellt, und wobei die vorstehend erwähnten Heterocylen zusätzlich durch eine Oxo-Gruppe substituiert sein können,
R^{c} ein Wasserstoffatom,
eine Hydroxygruppe,
eine C₁₋₃-Alkyloxygruppe,
eine Methoxygruppe, die durch ein bis drei Fluoratome substituiert ist,
eine Ethyloxygruppe, die in 2-Stellung durch einen Rest R⁵ substituiert ist, wobei
R⁵ eine Hydroxy-, C₁₋₃-Alkyloxy-, Amino-, C₁₋₃-Alkylamino-, Di-(C₁₋₃-alkyl)amino-, Bis-(2-methoxyethyl)-amino-, Pyrrolidin-1-yl-, Piperidin-1-yl- , Morpholin-4-yl-, Homomorpholin-4-yl-, Piperazin-1-yl- oder eine 4-C₁₋₃-Alkyl-piperazin-1-yl-Gruppe darstellt,
eine Propyloxygruppe, die in 3-Stellung durch den Rest R⁵ substituiert ist, wobei R⁵ wie vorstehend erwähnt definiert ist, oder
eine Butyloxygruppe, die in 4-Stellung durch einen Rest R⁵ substituiert ist, wobei R⁵ wie vorstehend erwähnt definiert ist, und
wobei, soweit nichts anderes erwähnt wurde, die vorstehend erwähnten Alkylgruppen geradkettig oder verzweigt sein können,
deren Tautomere, deren Stereoisomere, deren Gemische und deren Salze.

3. Bicyclische Heterocyclen der allgemeinen Formel 1 gemäß Anspruch 1, in denen
R^{a} eine 1-Phenylethyl-, 3-Ethinylphenyl-, 3-Brom-2-fluor-phenyl-, 3-Brom-4-fluorphenyl-, 3-Chlor-2-fluor-phenyl- oder 3-Chlor-4-fluor-phenyl-Gruppe,
eine 3-Chlor-4-benzyloxy-phenyl-, 3-Chlor-4-[(3-fluor-benzyl)oxy]-phenyl-, 4-(Pyridin-3-yloxy)-phenyl-, 4-[(6-Methyl-pyridin-3-yl)oxy]-phenyl-, 3-Methyl-4-(pyridin-3-yloxy)-phenyl-, 3-Methyl-4-[(6-methyl-pyridin-3-yl)oxy]-phenyl-, 3-Chlor-4-(pyridin-3-yloxy)-phenyl- oder 3-Chlor-4-[(6-methyl-pyridin-3-yl)oxy]-phenyl-Gruppe,
R^{b} eine Azetidin-1-yl-, Pyrrolidin-1-yl-, Piperidin-1-yl-, Morpholin-4-yl-, Piperazin-1-yl-, 4-(C₁₋₃-Alkyl-carbonyl)-piperazin-1-yl- oder 4-(C₁₋₃-Alkyl-sulfonyl)-piperazin-1-yl-Gruppe, die jeweils durch R⁴ mono- oder disubstituiert sein kann, wobei die Substituenten gleich oder verschieden sein können und
R⁴ ein Fluoratom,
eine C₁₋₃-Alkylgruppe,
eine Amino-, C₁₋₃-Alkylamino-, Di-(C₁₋₃-alkyl)amino-, C₁₋₃-Alkyl-carbonylamino-, N-(C₁₋₃-Alkyl)-C₁₋₃-alkyl-carbonylamino-, C₁₋₃-Alkyl-sulfonylamino- oder N-(C₁₋₃-Alkyl)-C₁₋₃-alkyl-sulfonylamino-Gruppe,
eine Amino-C₁₋₃-alkyl-, C₁₋₃-Alkylamino-C₁₋₃-alkyl-, Di-(C₁₋₃-alkyl)amino-C₁₋₃-alkyl-, C₁₋₃-Alkyl-carbonylamino-C₁₋₃-alkyl-, N-(C₁₋₃-Alkyl)-C₁₋₃-alkyl-carbonylamino-C₁₋₃-alkyl-, C₁₋₃-Alkyl-sulfonylamino-C₁₋₃-alkyl- oder N-(C₁₋₃-Alkyl)-C₁₋₃-alkyl- sulfonylamino-C₁₋₃-alkyl-Gruppe,
eine Hydroxy-, C₁₋₃-Alkyloxy- oder C₁₋₃-Alkyl-carbonyloxy-Gruppe
eine Hydroxy-C₁₋₃-alkyl-, C₁₋₃-Alkyloxy-C₁₋₃-alkyl- oder C₁₋₃-Alkyl-carbonyloxy-C₁₋₃-alkyl-Gruppe,
eine C₁₋₃-Alkyl-carbonyl-, Cyano-, C₁₋₄-Alkyl-oxycarbonyl-, Carboxy-, Aminocarbonyl-, C₁₋₃-Alkyl-aminocarbonyl- oder Di-(C₁₋₃-alkyl)amino-carbonyl-Gruppe,
eine C₁₋₃-Alkylcarbonyl-C₁₋₃-alkyl-, Cyano-C₁₋₃-alkyl-, C₁₋₄-Alkyloxycarbonyl-C₁₋₃-alkyl-gruppe, Aminocarbonyl-C₁₋₃-alkyl-, C₁₋₃-Alkylaminocarbonyl-C₁₋₃-alkyl- oder Di-(C₁₋₃-alkyl)aminocarbonyl-C₁₋₃-alkyl-Gruppe,
eine C₁₋₄-Alkylsulfanyl-, C₁₋₄-Alkylsulfinyl-, C₁₋₄-Alkylsulfonyl-, Aminosulfonyl-, C₁₋₃-Alkyl-aminosulfonyl- oder Di-(C₁₋₃-alkyl)amino-sulfonyl-Gruppe,
eine C₁₋₄-Alkylsulfanyl-C₁₋₃-alkyl-, C₁₋₄-Alkylsulfinyl-C₁₋₃-alkyl-, C₁₋₄-Alkylsulfonyl- C₁₋₃-alkyl-, Aminosulfonyl-C₁₋₃-alkyl-, C₁₋₃-Alkylaminosulfonyl-C₁₋₃-alkyl- oder Di-(C₁₋₃-alkyl)amino-sulfonyl-C₁₋₃-alkyl-Gruppe,
darstellt, und wobei die vorstehend erwähnten Heterocylen zusätzlich durch eine Oxo-Gruppe substituiert sein können,
R^{c} ein Wasserstoffatom,
eine Methoxy- oder Ethyloxy-Gruppe,
eine Ethyloxygruppe, die in 2-Stellung durch den Rest R⁵ substituiert ist, wobei
R⁵ eine Hydroxy-, Methoxy-, Ethoxy-, Amino-, Dimethylamino-, Diethylamino-, Bis-(2-methoxyethyl)-amino-, Pyrrolidin-1-yl-, Piperidin-1-yl-, Morpholin-4-yl-, Homomorpholin-4-yl-, Piperazin-1-yl-, 4-Methylpiperazin-1-yl- oder 4-Ethylpiperazin-1-yl-Gruppe darstellt,
eine Propyloxygruppe, die in 3-Stellung durch den Rest R⁵ substituiert ist, wobei R⁵ wie vorstehend erwähnt definiert ist, oder
eine Butyloxygruppe, die in 4-Stellung durch den Rest R⁵ substituiert ist, wobei R⁵ wie vorstehend erwähnt definiert ist, und
wobei, soweit nichts anderes erwähnt wurde, die vorstehend erwähnten Alkylgruppen geradkettig oder verzweigt sein können,
deren Tautomere, deren Stereoisomere, deren Gemische und deren Salze.

4. Bicyclische Heterocyclen der allgemeinen Formel I gemäß Anspruch 1, in denen
R^{a} eine 1-Phenylethyl-, 3-Ethinylphenyl-, 3-Brom-2-fluor-phenyl-, 3-Brom-4-fluor-phenyl-, 3-Chlor-2-fluor-phenyl- oder 3-Chlor-4-fluor-phenyl-Gruppe,
R^{b} eine Azetidin-1-yl-, Pyrrolidin-1-yl-, Piperidin-1-yl-, Morpholin-4-yl-, Piperazin-1-yl-, 4-(C₁₋₃-Alkyl-carbonyl)-piperazin-1-yl- oder 4-(C₁₋₃-Alkyl-sulfonyl)-piperazin-1-yl-Gruppe, die jeweils durch R⁴ mono- oder disubstituiert sein kann, wobei die Substituenten gleich oder verschieden sein können und
R⁴ ein Fluoratom,
eine C₁₋₃-Alkylgruppe,
eine Amino-, C₁₋₂-Alkylamino-, Di-(C₁₋₂-alkyl)amino-, C₁₋₂-Alkyl-carbonylamino-, N-(C₁₋₂-Alkyl)-C₁₋₂-alkyl-carbonylamino-, C₁₋₂-Alkyl-sulfonylamino- oder N-(C₁₋₂-Alkyl)-C₁₋₂-alkyl-sulfonylamino-Gruppe,
eine Amino-C₁₋₂-alkyl-, C₁₋₂-Alkylamino-C₁₋₂-alkyl-, Di-(C₁₋₂-alkyl)amino-C₁₋₂-alkyl-, C₁₋₂-Alkyl-carbonylamino-C₁₋₂-alkyl-, N-(C₁₋₂-Alkyl)-C₁₋₂-alkyl-carbonylamino-C₁₋₂-alkyl-, C₁₋₂-Alkyl-sulfonylamino-C₁₋₂-alkyl- oder N-(C₁₋₂-Alkyl)-C₁₋₂-alkyl-sulfonylamino-C₁₋₂-alkyl-Gruppe,
eine Hydroxy-, C₁₋₂-Alkyloxy- oder C₁₋₂-Alkyl-carbonyloxy-Gruppe,
eine Hydroxy-C₁₋₂-alkyl-, C₁₋₂-Alkyloxy-C₂₋₄-alkyl- oder C₁₋₂-Alkyl-carbonyloxy-C₁₋₂-alkyl-Gruppe,
eine C₁₋₂-Alkyl-carbonyl-, Cyano-, C₁₋₂-Alkyl-oxycarbonyl-, Carboxy-, Aminocarbonyl-, C₁₋₂-Alkyl-aminocarbonyl- oder Di-(C₁₋₂-alkyl)amino-carbonyl-Gruppe,
eine C₁₋₂-Alkylcarbonyl-C₁₋₂-alkyl-, Cyano-C₁₋₂-alkyl-, C₁₋₂-Alkyloxycarbonyl-C₁₋₂-alkyl-gruppe, Aminocarbonyl-C₁₋₂-alkyl-, C₁₋₂-Alkylaminocarbonyl-C₁₋₂-alkyl- oder Di-(C₁₋₂-alkyl)aminocarbonyl-C₁₋₂-alkyl-Gruppe,
eine C₁₋₂-Alkylsulfanyl-, C₁₋₂-Alkylsulfinyl- oder C₁₋₂-Alkylsulfonyl-Gruppe,
eine C₁₋₂-Alkylsulfanyl-C₁₋₂-alkyl-, C₁₋₂-Alkylsulfinyl-C₁₋₂-alkyl- oder C₁₋₂-Alkylsulfonyl-C₁₋₂-alkyl-Gruppe
darstellt, und wobei die vorstehend erwähnten Heterocylen zusätzlich durch eine Oxo-Gruppe substituiert sein können,
R^{c} ein Wasserstoffatom,
eine Methoxy-, Ethyloxy- oder 2-(Methoxy)-ethyloxy-Gruppe,
eine 2-(Morpholin-4-yl)ethyloxy-, 3-(Morpholin-4-yl)propyloxy- oder 4-(Morpholin-4-yl)butyloxy-Gruppe,
bedeuten,
deren Tautomere, deren Stereoisomere, deren Gemische und deren Salze.

5. Bicyclische Heterocyclen der allgemeinen Formel I gemäß Anspruch 1, in denen
R^{a} eine 1-Phenylethyl, 3-Ethinylphenyl-, 3-Chlor-2-fluor-phenyl- oder 3-Chlor-4-fluorphenyl-Gruppe,
R^{b} eine Azetidin-1-yl-, Pyrrolidin-1-yl-, Piperidin-1-yl-, Morpholin-4-yl-, Piperazin-1-yl-, 3-Oxo-piperazin-1-yl-, 4-Acetyl-piperazin-1-yl- oder 4-Methylsulfonyl-piperazin-1-yl-Gruppe, die jeweils durch R⁴ mono- oder disubstituiert sein kann, wobei die Substituenten gleich oder verschieden sein können und
R⁴ eine Methyl-, Hydroxy-, Methoxy-, Cyano-, Aminocarbonyl-, Methylaminocarbonyl- oder Dimethylamino-carbonyl-Gruppe darstellt, und
R^{c} ein Wasserstoffatom,
eine Methoxy-, Ethyloxy- oder 2-(Methoxy)-ethyloxy-Gruppe,
bedeuten,
deren Tautomere, deren Stereoisomere, deren Gemische und deren Salze.

6. Folgende Verbindungen der allgemeinen Formel I gemäß Anspruch 1:
(a) 4-[(3-Chlor-2-fluor-phenyl)amino]-6-[cis-4-(morpholin-4-yl)-cyclohexyloxy]-7-methoxy-chinazolin,
(b) 4-[(3-Chlor-2-fluor-phenyl)amino]-6-[trans-4-(morpholin-4-yl)-cyclohexyloxy]-7-methoxy-chinazolin,
(c) 4-[(3-Chlor-2-fluor-phenyl)amino]-6-[(R)-cis-4-(3-hydroxy-pyrrolidin-1-yl)-cyclohexyloxy]-7-methoxy-chinazolin,
(d) 4-[(3-Chlor-2-fluor-phenyl)amino]-6-[(R)-trans-4-(3-hydroxy-pyrrolidin-1-yl)-cyclohexyloxy]-7-methoxy-chinazolin,
(e) 4-[(3-Chlor-2-fluor-phenyl)amino]-6-[(S)-cis-4-(3-hydroxy-pyrrolidin-1-yl)-cyclohexyloxy]-7-methoxy-chinazolin,
(f) 4-[(3-Chlor-2-fluor-phenyl)amino]-6-[(S)-trans-4-(3-hydroxy-pyrrolidin-1-yl)-cyclohexyloxy]-7-methoxy-chinazolin,
(g) 4-[(3-Chlor-2-fluor-phenyl)amino]-6-[cis-4-(3-oxo-piperazin-1-yl)-cyclohexyloxy]-7-methoxy-chinazolin,
(h) 4-[(3-Chlor-2-fluor-phenyl)amino]-6-[trans-4-(3-oxo-piperazin-1-yl)-cyclohexyloxy]-7-methoxy-chinazolin,
(i) 4-[(3-Chlor-2-fluor-phenyl)amino]-6-{(S)-cis-4-[2-(N,N-dimethylaminocarbonyl)-pyrrolidin-1-yl]-cyclohexyloxy]-7-methoxy-chinazolin,
(j) 4-[(3-Chlor-2-fluor-phenyl)amino]-6-{(S)-trans-4-[2-(N,N-dimethylaminocarbonyl)- pyrrolidin-1-yl]-cyclohexyloxy]-7-methoxy-chinazolin,
(k) 4-[(3-Chlor-2-fluor-phenyl)amino]-6-{(S)-cis-4-[2-(aminocarbonyl)-pyrrolidin-1-yl]-cyclohexyloxy]-7-methoxy-chinazolin und
(l) 4-[(3-Chlor-2-fluor-phenyl)amino]-6-{(S)-trans-4-[2-(aminocarbonyl)-pyrrolidin-1-yl]-cyclohexyloxy]-7-methoxy-chinazolin,
sowie deren Salze.

7. Physiologisch verträgliche Salze der Verbindungen nach mindestens einem der Ansprüche 1 bis 6 mit anorganischen oder organischen Säuren oder Basen.

8. Arzneimittel, enthaltend eine Verbindung nach mindestens einem der Ansprüche 1 bis 6 oder ein physiologisch verträgliches Salz gemäß Anspruch 7 neben gegebenenfalls einem oder mehreren inerten Trägerstoffen und/oder Verdünnungsmitteln.

9. Verwendung einer Verbindung nach mindestens einem der Ansprüche 1 bis 7 zur Herstellung eines Arzneimittels, das zur Behandlung von benignen oder malignen Tumoren, zur Vorbeugung und Behandlung von Erkrankungen der Atemwege und der Lunge sowie zur Behandlung von Erkrankungen des Magen-Darm-Traktes und der Gallengänge und -blase geeignet ist.

10. Verfahren zur Herstellung eines Arzneimittels gemäß Anspruch 8, **dadurch gekennzeichnet, daß** auf nicht-chemischem Wege eine Verbindung nach mindestens einem der Ansprüche 1 bis 7 in einen oder mehrere inerte Trägerstoffe und/oder Verdünnungsmittel eingearbeitet wird.

11. Verfahren zur Herstellung der Verbindungen der allgemeinen Formel I gemäß den Ansprüchen 1 bis 6, **dadurch gekennzeichnet, dass**
a) eine Verbindung der allgemeinen Formel 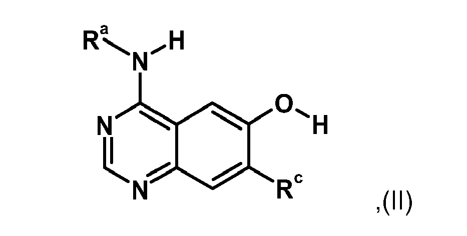 in der
R^{a} und R^{c} wie eingangs erwähnt definiert sind, mit einer Verbindung der allgemeinen Formel 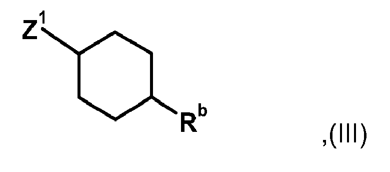 in der
R^{b} wie eingangs erwähnt definiert ist und Z¹ eine Austrittsgruppe darstellt, umgesetzt wird, oder
b) eine Verbindung der allgemeinen Formel 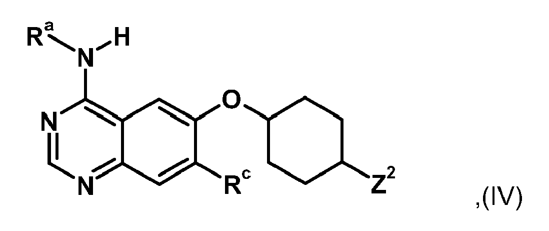 in der
R^{a} und R^{c} wie eingangs erwähnt definiert sind, und Z² eine Austrittsgruppe darstellt, mit einer Verbindung der allgemeinen Formel
H-R^{b} ,(V)
in der
R^{b} wie eingangs erwähnt definiert ist, umgesetzt wird, oder
c) eine Verbindung der allgemeinen Formel 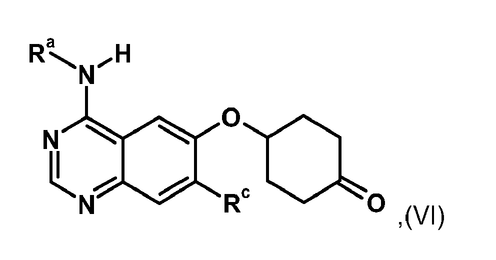 in der
R^{a} und R^{c} wie eingangs erwähnt definiert sind, mit einer Verbindung der allgemeinen Formel
H-R^{b} ,(VII)
in der
R^{b} wie eingangs erwähnt definiert ist, umgesetzt wird, oder
d) eine Verbindung der allgemeinen Formel (VIII) 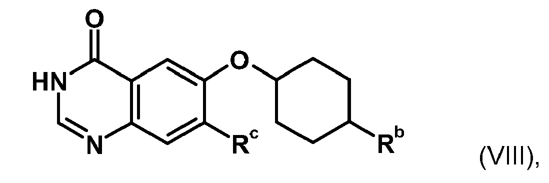 in der R^{b} und R^{c} wie eingangs erwähnt definiert sind, mit einem Halogenierungsmittel zu einer Zwischenverbindung der allgemeinen Formel (IX), 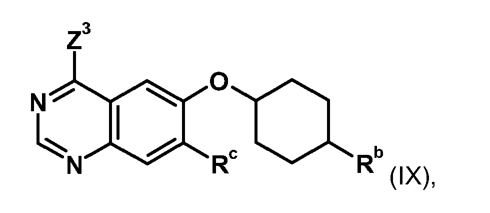 in der R^{b} und R^{c} wie eingangs erwähnt definiert sind und Z³ ein Halogenatom darstellt, umgesetzt wird, und diese anschließend mit einer Verbindung der allgemeinen Formel (X),
R^{a}-NH₂ (X), in der R^{a} wie eingangs erwähnt definiert ist, umgesetzt wird.
e) Zur Herstellung von Verbindungen der allgemeinen Formel I, in der R^{c} eine der eingangs erwähnten, gegebenenfalls substituierten Alkyloxygruppen darstellt,
eine Verbindung der allgemeinen Formel 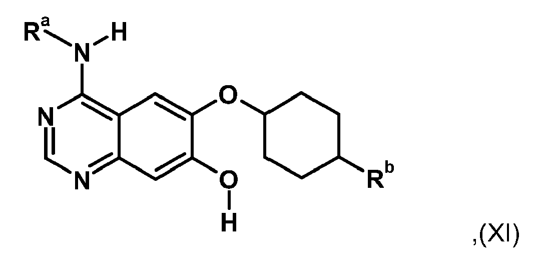 in der R^{a} und R^{b} wie eingangs erwähnt definiert sind, mit einer mit einer Verbindung der allgemeinen Formel
Z⁴-R^{c'} ,(XII)
in der R^{c'} wie eingangs erwähnt definiert ist und Z⁴ eine Austrittsgruppe darstellt, umgesetzt wird, oder
f) Zur Herstellung von Verbindungen der allgemeinen Formel I, in der R^{c} eine der eingangs erwähnten Alkyloxygruppen darstellt, die durch eine gegebenenfalls substituierte Amino-, Alkylamino- oder Dialkylaminogruppe oder durch eine gegebenenfalls substituierte, über ein Iminostickstoffatom gebundene heterocyclischen Gruppe substituiert ist, eine Verbindung der allgemeinen Formel 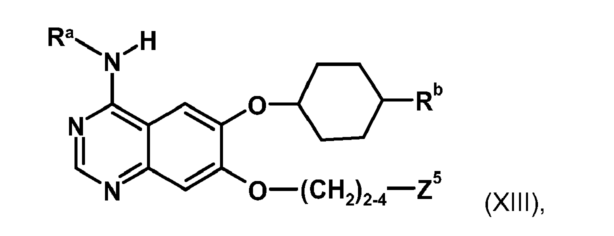 in der R^{a} und R^{b} wie eingangs erwähnt definiert sind und Z⁵ eine Austrittsgruppe darstellt, mit Ammoniak, einem entsprechenden, gegebenenfalls substituierten Alkylamin, Dialkylamin oder einer Iminoverbindung oder deren geeigneten Salzen oder Derivaten, umgesetzt wird, oder
g) Zur Herstellung von Verbindungen der allgemeinen Formel 1, in der R^{b} eine oder mehrere Hydroxygruppen enthält,
Schutzreste von einer Verbindung der allgemeinen Formel 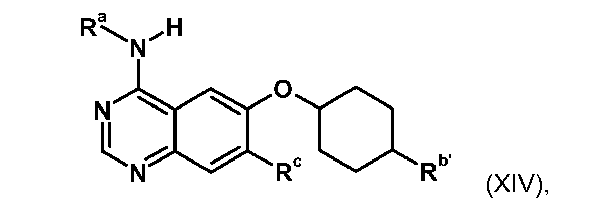 in der R^{a} und R^{c} wie eingangs erwähnt definiert sind und R^{b'} eine oder mehrere in Hydroxygruppen überführbare Gruppen enthält, abgespalten werden, oder
h) Zur Herstellung von Verbindungen der allgemeinen Formel I, in der R^{b} eine -NH-Gruppe enthält:
ein Schutzrest von einer Verbindung der allgemeinen Formel 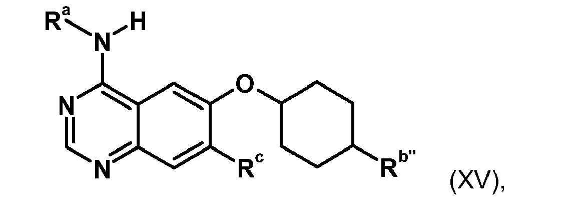
in der R^{a} und R^{c} wie eingangs erwähnt definiert sind und R^{b"} mit der Maßgabe die eingangs für R^{b} erwähnten Bedeutungen besitzt, daß R^{b"} ein geschütztes Stickstoffatom enthält, abgespalten wird, und
gewünschtenfalls eine so erhaltene Verbindung der allgemeinen Formel I, die eine Amino-, Alkylamino- oder Iminogruppe enthält, mittels Acylierung oder Sulfonylierung in eine entsprechende Acyl- oder Sulfonylverbindung der allgemeinen Formel I übergeführt wird, und/oder
eine Verbindung der allgemeinen Formel I, die eine Amino-, Alkylamino- oder Iminogruppe enthält, mittels Alkylierung oder reduktiver Alkylierung in eine entsprechende Alkylverbindung der allgemeinen Formel I übergeführt wird und/oder
eine Verbindung der allgemeinen Formel I, die eine Alkoxycarbonylgruppe enthält, durch Esterspaltung in eine Carbonsäure übergeführt wird, und/oder
eine Verbindung der allgemeinen Formel I, die eine Alkoxycarbonylgruppe enthält, durch Umsetzung mit einem Amin in ein Carbonsäureamid-Derivat übergeführt wird, und/oder
eine Verbindung der allgemeinen Formel I, die eine Carboxy-Gruppe enthält, durch Umsetzung mit einem Amin in ein Carbonsäureamid-Derivat übergeführt wird, und/oder
erforderlichenfalls ein bei den vorstehend beschriebenen Umsetzungen verwendeter Schutzrest wieder abgespalten wird und/oder
gewünschtenfalls eine so erhaltene Verbindung der allgemeinen Formel I in ihre Stereoisomere aufgetrennt wird und/oder
eine so erhaltene Verbindung der allgemeinen Formel I in ihre Salze, insbesondere für die pharmazeutische Anwendung in ihre physiologisch verträglichen Salze, übergeführt wird.
